# EUROPEAN PATENT SPECIFICATION

(11) **EP 2 519 279 B1**
(45) Date of publication and mention of the grant of the patent: **22.04.2015**
(21) Application number: 10798334.8
(22) Date of filing: 22.12.2010
(51) Int. Cl.: A61M 1/36

(54) **DEVICE AND METHOD FOR MONITORING A FLUID FLOW RATE IN A CARDIOVASCULAR SYSTEM**
VORRICHTUNG UND VERFAHREN ZUR ÜBERWACHUNG EINER STRÖMUNGSRATE IN EINEM HERZ-KREISLAUF-SYSTEM
DISPOSITIF ET PROCÉDÉ DE CONTRÔLE DE LA VITESSE D'ÉCOULEMENT DES FLUIDES DANS UN SYSTÈME CARDIOVASCULAIRE

(30) Priority: 28.12.2009 SE 0951030; 28.12.2009 US 290319 P
(43) Date of publication of application: 07.11.2012
(73) Proprietor: Gambro Lundia AB, 220 10 Lund (SE)
(72) Inventor: OLDE, Bo, SE-224 67 Lund (SE); SOLEM, Kristian, SE-244 31 Kävlinge (SE)
(86) International application number: PCT/EP2010/070565
(87) International publication number: WO 2011/080194

(56) References cited:
- WO-A1-2004/067064
- WO-A2-02/053025
- DE-C1- 19 901 078
- US-A- 5 178 603
- US-A- 5 830 365
- US-A1- 2008 108 930

## Description

### Technical field

The present invention relates to a device and method for monitoring fluid flow rates in a subject's cardio-vascular system. More particularly, the invention relates to the calculation of flow rates in the cardio-vascular system based on temporal signal profiles of pulses originating from a pulse generator of the subject.

### Background art

In extracorporeal blood treatment, blood is taken out of a subject, treated and then reintroduced into the subject by means of an extracorporeal blood flow circuit. Generally, the blood is circulated through the circuit by one or more pumping devices. The circuit is connected to a blood vessel access of the patient, typically via one or more access devices, such as needles, which are inserted into the blood vessel access. Such extracorporeal blood treatments include hemodialysis, hemodiafiltration, hemofiltration, plasmapheresis, bloodbanking, blood fraction separation (e.g. cells) of donor blood, etc.

In hemodialysis and similar treatments, a blood access is commonly surgically created in the nature of an arterio-venous (AV) shunt, commonly referred to as a fistula. Blood needles or catheters are inserted into the fistula. Blood is taken out from the fistula via a needle or catheter at an upstream position and blood is returned to the fistula via a needle or catheter at a downstream position. The arterio-venous shunt or fistula provides a blood access having capability of providing a high blood flow and being operative during several years and even tens of years. It is produced by connecting, for example, the radial artery to the cephalic vein at the level of the forearm. The venous limb of the fistula thickens during the course of several months, permitting repeated insertion of dialysis needles or catheters. Alternative blood accesses to the fistula are for instance an arterio-venous graft or a silicon, dual-lumen catheter surgically implanted into one of the large veins. Other methods and devices are also known.

During the above blood treatment therapies, hemodialysis for instance, it is desirable to obtain a constant blood flow rate of 150 - 500 ml/min or even higher, and the access site must be prepared for delivering such flow rates. The blood flow in an AV fistula is often 800 ml/min or larger, permitting delivery of a blood flow rate in the desired range.

In the absence of a sufficient treated blood entering the fistula via the venous needle, the extracorporeal circuit blood pump will take up some of the already treated blood entering the fistula via the venous needle, so called access or fistula recirculation, leading to poor treatment results and progressive reduction of treatment efficiency.

A common cause of poor flow with AV fistulas is partial obstruction of the venous limb due to fibrosis secondary to multiple veinpunctures. Moreover, stenosis causes a reduction of access flow.

It is known that access flow rate often exhibits a long plateau time period with sufficient access flow, followed by a short period of a few weeks with markedly reduced access flow leading to recirculation and ultimately access failure. By monitoring the evolution of the access flow during consecutive treatment sessions, it is possible to detect imminent access flow problems. Proper detection of access flow reduction may help in carrying out a maintenance procedure on the access thereby avoiding any access failure.

It is known in the art to measure different parameters of a cardiovascular system. In particular, methods are known for detection of parameters relating to the access, such as access flow, or to cardiac output.

Monitoring of parameters such as access flow of a blood access such as a fistula may be performed by ultrasound (Doppler) measurements. Alternatively, dilution methods may be used to obtain access flow by measuring a difference in conductivity created by a reversal of flow directions of blood to and from the blood access.

However, known methods for determining flow-related parameters during dialysis or blood treatment require separate, specialised instruments and sensors, and are not suited for repeated or continuous, beat-to-beat measurements and monitoring.

In particular, the known methods comprise isolated measurements, and are not well-suited for continuous intra-dialytic monitoring of cardiac output and access flow.

Another technique for estimating the blood flow rate in the vascular access of a patient undergoing extracorporeal blood treatment is proposed in WO2004/067064. According to embodiments of this technique the static pressures in the arterial and venous blood lines of an extracorporeal blood circuit are measured, by pressure sensors in the extracorporeal blood circuit, while the flow rate of a blood pump in the extracorporeal blood circuit is varied. The blood flow rate in the vascular access is calculated using a mathematical relation that describes the pressure in the vascular access as a function of the static pressures in the arterial and venous blood lines, and a mathematical relation that describes the pressure in the vascular access as a function of the blood flow rate therein. The calculated blood flow rate represents an average of the blood flow rate in the vascular access. Thus, the technique proposed in WO2004/067064 is not suited for continuous, beat-to-beat measurements and monitoring, and it is not implemented to detect pressure pulses that originate from a pulse generator in the patient, such as pressure pulses caused by heartbeats or the breathing of the patient, or to estimate the blood flow rate based on such pressure pulses.

DE19901078 discloses a technique for detecting stenosis in a blood access circuit (graft, fistula) or in an extracorporeal circuit by monitoring the amplitude of pressure pulses detected by a pressure sensor in an extracorporeal circuit. There is no suggestion in DE19901078 that the flow rate through the blood access circuit could be calculated based on the pressure pulses.

Enhanced monitoring of cardio-vascular flows may provide numerous benefits, in particular in connection with extracorporeal treatments. For instance, monitoring of cardiac output would be beneficial in connection with dialysis since water removal, i.e. ultrafiltration, during dialysis may reduce cardiac output, which may lead to an increased risk for the subject undergoing the treatment to suffer from hypotension. The reason is that cardiac output depends on the venous blood flow returning to the heart, which in turn may decrease as the total blood volume decreases after running ultrafiltration at higher rate compared to the vascular refill rate.

Access flow measurements are important for the clinician to determine if a blood access of a dialysis patient can provide sufficient blood flow to allow adequate dialysis treatment. Normally, access flow measurements are conducted regularly, e.g. once a month, in order to detect low values or declining trends. Such indications may urge the physician to perform an access intervention for instance surgery to alleviate the situation.

Hence, there is a need for monitoring various cardiovascular flows, in particular during extracorporeal treatment such as dialysis.

Continuous cardiac output measurement may be important in adjusting the ultrafiltration rate properly to reduce the risk for hypotension.

In addition, cardiac output variations between treatments or over longer periods may be an indication of a heart condition, which may call for further medical investigation.

### Summary of the Invention

It is an object of the invention to at least partly overcome one or more of the above-identified limitations of the prior art and in particular to provide an alternative or complementary technique for monitoring fluid flow rates in a subject's cardio-vascular system. Specifically, it is an object to provide calculations of flow rates in the cardio-vascular system based on temporal signal profiles of pulses originating from a pulse generator of the subject.

This and other objects, which will appear from the description below, are at least partly achieved by means of a device for monitoring and a method for monitoring according to the independent claims, embodiments thereof being defined by the dependent claims.

A first aspect of the invention is a device for monitoring a fluid flow rate of a cardiovascular system of a subject, said device comprising an input for obtaining a time-dependent measurement signal from a pressure sensor in an extracorporeal blood circuit which is adapted for connection to the cardiovascular system, the pressure sensor being arranged to detect a subject pulse originating from a subject pulse generator, wherein the device further comprises a signal processor connected to the input and being configured to: process the measurement signal to obtain a pulse profile which is a temporal signal profile of the subject pulse, and calculate the fluid flow rate based at least partly on the temporal signal profile.

In one embodiment, the pulse generator is a part of the cardiovascular system. Hence, requiring no external source.

In one embodiment, the pulse generator is any of the heart, the breathing system, or any combinations thereof. Hence, the pulse generator is part of the cardiovascular system.

In one embodiment, the extracorporeal blood circuit comprises a fluid pathway, a blood processing device, and at least one pumping device, and wherein the pressure sensor is further arranged to detect a pump pulse originating from the pumping device.

In one embodiment, the calculating of the fluid flow rate (Q) involves a pulse parameter P from one or more of amplitude, shape, and timing of the temporal signal profile. The pulse parameter P may also be referred to as a pulse feature.

A second aspect of the invention is a method for monitoring a fluid flow rate in a cardiovascular system of a subject, said method comprising: obtaining a time-dependent measurement signal from a pressure sensor in an extracorporeal blood circuit which is arranged in fluid connection with the cardiovascular system, the pressure sensor being arranged to detect a subject pulse originating from a subject pulse generator; processing the measurement signal to obtain a pulse profile which is a temporal signal profile of the subject pulse, and calculating the fluid flow rate based at least partly on the temporal signal profile.

In one embodiment, the method further comprises varying a blood flow of the extracorporeal circuit. Hence, by varying the blood flow in an extracorporeal circuit of for instance a dialysis monitor to cause a perturbation to the hydraulic system in the fistula such that the dynamics in the hydraulic system is altered and thus increasing the number of relationships for determining the number of unknown variables. The variation may be caused by for instance varying the direction and/or magnitude of the blood flow.

In one embodiment, the method further comprises aggregating a plurality of subject pulse profiles within an aggregation time window in the measurement signal and calculating the fluid flow rate based on an average of the plurality of the subject pulse profiles.

In one embodiment, the calculating involves calculation of the cardiac output of the cardiovascular system. An alarm event may be generated when the Cardiac Output (CO) exceeds a predetermined threshold.

In one embodiment, the calculating involves calculation of an access flow of a blood access in the cardiovascular system.

In one embodiment, the method further comprises calibrating the fluid flow rate against one or more calibration values. For instance, the method may be calibrated with one or more reference values at the beginning of and/or prior to a treatment, at the end of a treatment and/or during a treatment.

In one embodiment, the method further comprises calculating an average access flow rate Qa and an associated variability QaV, retrieving a withdrawal blood flow rate Qb, and in case the sum of blood flow rate Qb and access flow variance QaV exceeds Qa, generating an alarm event. Hence, recirculation may be detected. The variability QaV may comprise e.g. standard deviation or variance. Alternatively, an alarm event may be generated in a case when the withdrawal blood flow rate Qb exceeds a momentary access flow rate Qa.

In one embodiment, the method further comprises calculating at least one additional fluid flow rate (Qx), calculating an an average fluid flow rate (Qavg) determined from the calculated fluid flow rate (Q) and the at least one additional fluid flow rate (Qx), calculating an average reference fluid flow rate (Qavg_ref), and adjusting the average reference fluid flow rate (Qavt_ref) based on the fluid flow rate (Q) and the at least oen additional fluid flow rate (Qx). Hence, reference measurements of average fluid flow rates may be corrected by taking into account the variance of the fluid flow rate, i.e. fluctuations or periodical variations.

In one embodiment, the calibration comprises: providing a detectable perturbation to at least a measurable blood characteristic in the cardiovascular system; measuring an integrated change of a corresponding characteristic on a treatment fluid outlet of the extracorporeal blood circuit; and determining the cardiovascular flow rate based on the measurement of said integrated change of the treatment fluid outlet. For instance, the detectable perturbation may be in the form of a pulse. The perturbation may be a change in concentration of a detectable substance such as urea, salt or salt in the form of a saline solution.

In one embodiment, the calibration comprises: obtaining a first conductivity or concentration measurement in a treatment fluid of the extracorporeal blood circuit running in a first direction; obtaining a second conductivity or concentration measurement in the treatment fluid running in a second direction, and calculating the access flow rate in said blood access as a function of: said first conductivity or concentration measurement and of said second conductivity or concentration measurement.

In one embodiment, the method further comprises calculating a reference fluid flow rate (Qref) at a time point when the calculated fluid flow rate (Q) corresponds to an average fluid flow rate. In this way, reference measurements are determined at instances of average fluid flow rates, suppressing effects from variability in the fluid flow rate.

In one embodiment, the method further comprises: defining an initial model; assigning the initial model to a current model; generating a parameter that correlates with the fluid flow rate; acquiring flow calibration data; investigating whether a model validity criterion is fulfilled or not by comparing parameter, calibration data with the current model, wherein in case the model validity criterion is not fulfilled then repeatedly generating a new model and assigning the current model with new model until model validity criterion is fulfilled; wherein in case the model validity criterion is fulfilled, calculating a fluid flow rate based at least partly on the temporal signal profile.

In one embodiment, the method further comprises one or more of acquiring blood pressure of the subject and comparing said blood pressure with the current model; and storing of current model and available parameters.

In one embodiment, the calculating of a fluid flow rate (Q) involves a pulse parameter P from one or more of amplitude, shape, and timing of the temporal signal profile. The pulse parameter P may also be referred to as a pulse feature.

The calculated fluid flow rate may for instance be the cardiac output of the cardiovascular system of the subject or the access flow of a blood access in the cardiovascular system.

The signal processor of the device of the first aspect of the invention is further configured to carry out any of the steps of the methods according to the second aspect of the invention.

Embodiments of the second aspect of the invention may correspond to the above-identified embodiments of the first aspect of the invention.

Still other objectives, features, aspects and advantages of the present invention will appear from the following detailed description, from the attached claims as well as from the drawings.

### Brief Description of the Drawings

Embodiments of the inventive concepts will now be described in more detail with reference to the accompanying schematic drawings.
Fig. 1 is a schematic view of a general fluid arrangement in which the inventive concepts may be used for monitoring a fluid flow rate in a cardiovascular system.
Fig. 2 is a partially schematic view of a forearm of a subject provided with an arterial/venous (AV) fistula.
Fig. 3 is a schematic view of a system for hemodialysis treatment including an extracorporeal blood flow circuit.
Fig. 4 (a) is a plot in the time domain of a venous pressure signal containing both pump frequency components and a heart signal, and Fig. 4(b) is a plot of the corresponding signal in the frequency domain.
Fig. 5 is a flow chart of a monitoring process according to an embodiment of the invention.
Fig. 6(a) is a plot of a pressure signal as a function of time, and Fig. 6(b) is a plot of the pressure signal after filtering.
Fig. 7 is a plot of cardiac output variations seen decreasing during a treatment.
Fig. 8 is a plot of cardiac output variations seen stable during a treatment.
Fig. 9 is a flow chart of a signal analysis process according to an embodiment of the invention.
Fig. 10 is a flow chart of a monitoring process according to an embodiment of the invention.
Fig. 11 is a block diagram of a hydraulic model of a cardiovascular system according to the present invention.
Fig. 12 is an enlargement view of the block diagram of Fig. 11.
Fig. 13 is a plot of access flow variations obtained with ultrasound measurement.
Fig. 14 is a plot showing a subject pulse parameter according to one embodiment of the invention.
Fig. 15 is a plot of fistula pressure modelled based on the heart pulse.
Fig. 16 is a plot of two phases of a heart pulse wave form illustrating one aspect of the invention.

### Detailed Description of Example Embodiments

In the following, different embodiments for monitoring a fluid flow rate in a cardiovascular system, in particular cardiac output and access flow, will be described with reference to an exemplifying circuit for extracorporeal blood treatment.

Throughout the following description, like elements are designated by the same reference signs.

It has surprisingly been found that time-dependent pressure measurements of pulses in an extracorporeal blood circuit coupled to the cardiovascular system of a subject may reveal vital information of fluid flow rates in the cardiovascular system, such as cardiac output and access flow in for instance a fistula. The pressure pulses have origin in a pulse generator, for instance a physiological such as the heart, breathing system, or a non-physiological such as an external pulse generator inducing pulses into the cardiovascular system, such as by inducing pressure puffs with a blood pressure cuff.

In the text below, the "subject pulse" may refer to any one of the following: heart pulse or breathing pulse.

### I. GENERAL

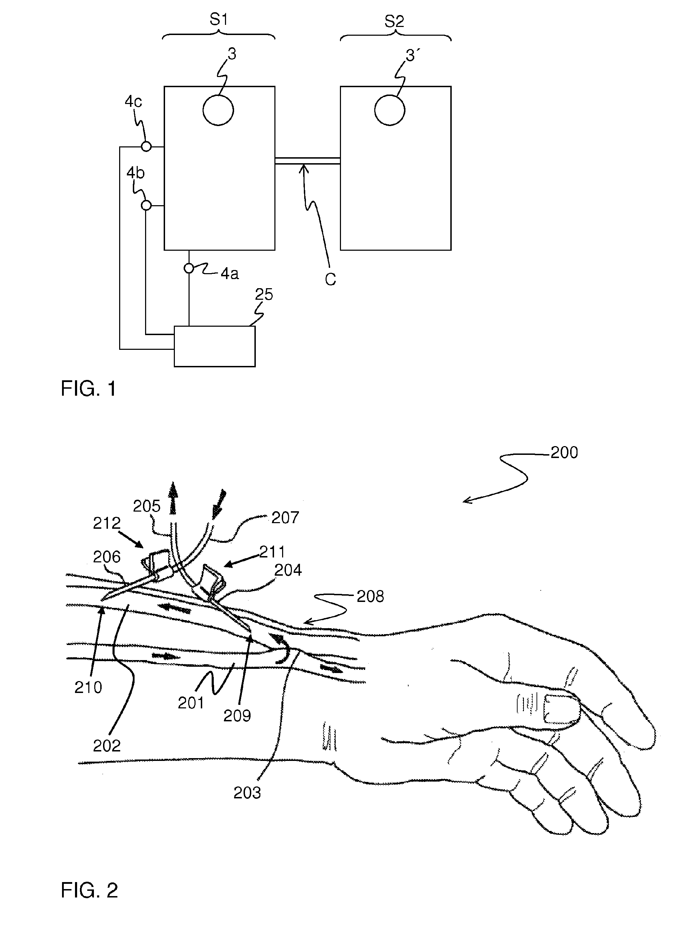

Fig. 1 illustrates a general fluid arrangement in which a fluid connection C is established between a first fluid containing system S1 and a second fluid containing system S2. The fluid connection C may or may not transfer fluid from one system to the other. A first pulse generator 3, e.g. pump, is arranged to generate a series of pressure waves in the fluid within the first system S1, and a second pulse generator 3', e.g. a subject pulse generator such as the heart or breathing, is arranged to generate a series of pressure waves in the fluid within the second system S2. Pressure sensors 4a and 4c are arranged to measure the fluid pressure in the first system S1. As long as the fluid connection C is intact, pressure waves generated by the second pulse generator 3' will travel from the second system S2 to the first system S1, and thus second pulses originating from the second pulse generator 3' will be detected by the pressure sensors 4a and 4c in addition to first pulses originating from the first pulse generator 3. It is to be noted that either one of the first and second pulse generators 3, 3' may include more than one pulse-generating device. Further, any such pulse-generating device may or may not be part of the respective fluid containing system S1, S2.

As used herein, a "pressure wave" denotes a mechanical wave in the form of a disturbance that travels or propagates through a material or substance. The pressure waves typically propagate in the fluid at a velocity of about 3-20 m/s. The pressure sensor generates measurement data that forms a pressure pulse for each pressure wave. A "pressure pulse" or "pulse" is thus a set of data samples that define a local increase or decrease (depending on implementation) in signal magnitude within a time-dependent measurement signal ("pressure signal"). The pressure pulses appear at a rate proportional to the generation rate of the pressure waves at the pulse generator. The pressure sensor may be of any type, e.g. operating by resistive, capacitive, inductive, magnetic or optical sensing, and using one or more diaphragms, bellows, Bourdon tubes, piezo-electrical components, semiconductor components, strain gauges, resonant wires, photo-plethysmography (PPG), accelerometers, bioimpedance, etc.

The fluid arrangement of Fig. 1 further includes a surveillance device 25 which is connected any one of the pressure sensors 4a-4c, as indicated in Fig. 1. Thereby, the surveillance device 25 acquires one or more measurement signals that are time-dependent to provide a real time representation of the fluid pressure in the first system S1. The surveillance device 25 monitors a vascular flow rate of the cardiovascular system of the subject, based on the principle that characteristics, such as magnitude, shape and/or phase, of the second pulses vary depending on the status of the cardiovascular system. Characteristics in the second pulses are related to the characteristics of fluid flows in the cardiovascular system, and these characteristics may be collected for subsequent analysis or comparison over time of how the various fluid flows develop and change.

The surveillance device 25 is thus configured to continuously process the time-dependent measurement signal(s) to monitor one or more vascular flows, such as the cardiac output (CO) or access flow Qₐ. Typically, the determination involves analyzing the measurement signal(s), or a pre-processed version thereof, in the time domain to calculate a value of an evaluation parameter which is indicative of the characteristics of the second pulses in the measurement signal(s). Depending on implementation, the surveillance device 25 may use digital components or analogue components, or a combination thereof, for receiving and processing the measurement signal(s).

### II. EXAMPLE OF AN EXTRACORPOREAL CIRCUIT

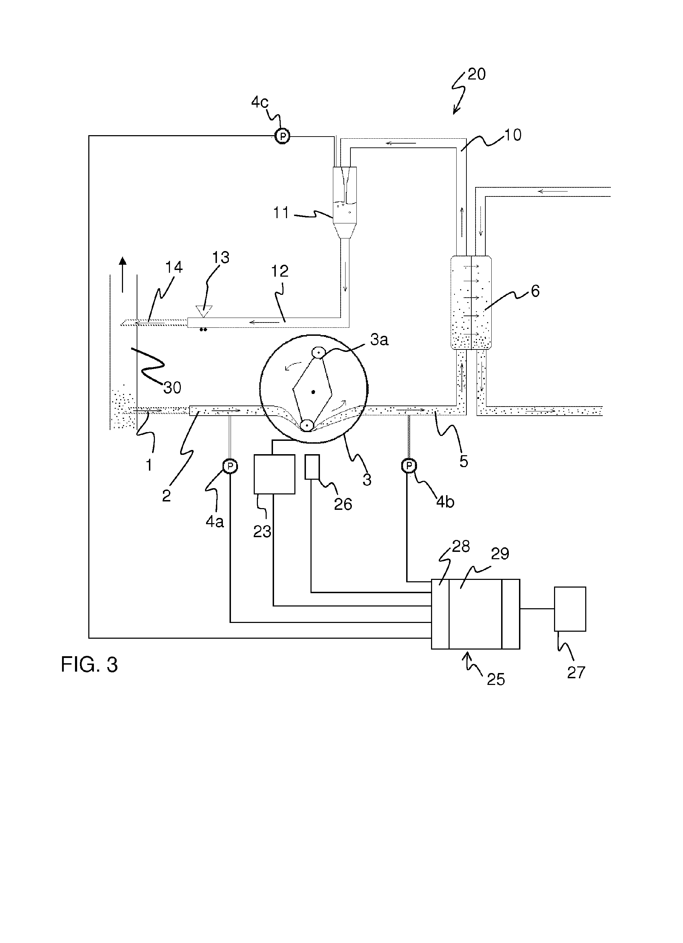

Fig. 3 exemplifies the first system S1 of Fig. 1 in the form of an extracorporeal blood flow circuit 20 of the type which is used for dialysis. The second system S2 of Fig. 1 corresponds to a subject indicated in Fig. 3 by blood vessel 30. Thus, the extracorporeal blood flow circuit 20 comprises an access device for blood extraction in the form of an arterial needle 1, and an arterial tube segment 2 which connects the arterial needle 1 to a blood pump 3 which may be of peristaltic type, as indicated in Fig. 3. Withdrawal or artery needle 1 and return or venous needle 14 are shown connected to a vessel 30 of the subject, which vessel is a part of the cardio-vascular system of the subject. At the inlet of the pump 3 there is a pressure sensor 4a, hereafter referred to as arterial sensor, which measures the pressure before the pump in the withdrawal tube segment 2. The blood pump 3 propels the blood from the fistula, through the withdrawal needle 1, via a pre-dialyser tube segment 5, to the blood-side of a dialyser 6. Many dialysis machines are additionally provided with a pressure sensor 4b that measures the pressure between the blood pump 3 and the dialyser 6. The blood is lead via a post-dialyser tube segment 10 from the blood-side of the dialyser 6 to a venous drip chamber or deaeration chamber 11 and from there back to the subject via the return tube segment 12 and return needle 14. A pressure sensor 4c, hereafter referred to as venous sensor, is provided to measure the pressure on the venous side of the dialyser 6. In the illustrated example, the pressure sensor 4c measures the pressure in the venous drip chamber. Both the withdrawal needle 1 and the return needle 14 are connected to the subject by means of the vascular access.

As discussed by way of introduction, it may be vital to monitor the fluid flows in a cardiovascular system with respect to variations, levels and/or changes. In many dialysis monitors, one or more of said pressure detectors 4a-4c are not present. However, there will be at least one venous pressure sensor. The following description is focused on monitoring various vascular flows, with specific examples relating to the cardiac output (CO) and access flow Qₐ based on a measurement signal from one or more of the pressure sensors.

Further in Fig. 3, a control unit 23 is provided, i.a., to control the blood flow in the circuit 20 by controlling the revolution speed of the blood pump 3. The extracorporeal blood flow circuit 20 and the control unit 23 may form part of an apparatus for extracorporeal blood treatment, such as a dialysis machine. Although not shown or discussed further it is to be understood that such an apparatus performs many other functions, e.g. controlling the flow of dialysis fluid, controlling the temperature and composition of the dialysis fluid, etc.

Also in Fig. 3, a surveillance device 25 is configured to monitor various vascular flows, with specific examples relating to the cardiac output (CO) and access flow Qₐ, by analysing pressure response for instance originating from the subject's heart in a blood pressure pulse signal. The surveillance device 25 is connected to receive a measurement signal from any of the pressure sensors 4a-4c. The device 25 may also be connected any additional pressure sensors included in the extracorporeal blood flow circuit 20. As indicated in Fig. 3, the device 25 may also be connected to the control unit 23. Alternatively or additionally, the device 25 may be connected to a pump sensor 26, such as a rotary encoder (e.g. conductive, optical or magnetic) or the like, for indicating the frequency and phase of the blood pump 3. The surveillance device 25 is tethered or wirelessly connected to a local or remote device 27 for continuously presenting updated values of one or more of the determined vascular flows on a screen, storing on a memory and/or generating an audible/visual/tactile alarm or warning signal in case of one or more vascular flows of the cardiovascular system falls below acceptable levels. For instance, occurrence of recirculation may be detected. In order to avoid recirculation, i.e. treated blood return re-enters the withdrawal line, the average or momentary access flow rate Qa should be greater than the sum of blood flow rate Qb withdrawn from the access and a component relating to variability in the access flow. The surveillance device 25 and/or the remote device 27 may alternatively be incorporated as part of a dialysis monitor.

Additionally, in Fig. 3, the surveillance device 25 comprises a data acquisition part 28 for pre-processing the incoming signal(s), e.g. including an A/D converter with a required minimum sampling rate and resolution, one or more signal amplifiers, one or more filters to remove undesired components of the incoming signal(s), such as offset, high frequency noise and supply voltage disturbances.

In the examples given herein, the data acquisition part 28 comprises a DAQ card USB-6210 from National Instruments with a sampling rate of 1 kHz and resolution of 16 bits, an operation amplifying circuit AD620 from Analogue Devices, a high-pass filter with a cut-off frequency of 0.03 Hz (i.a., for removal of signal offset) together with a low-pass filter with a cut-off frequency of 402 Hz (i.a., for removal of high frequency noise). To obtain a short convergence time, a low-order filter is used for the high-pass filter. Furthermore, the data acquisition part 28 may include an additional fixed band-pass filter with upper and lower cut-off frequencies of 0.5 Hz and 2.7 Hz, respectively, which corresponds to heart pulse rates between 30 and 160 beats per minute. This filter may be used to suppress disturbances outside the frequency interval of interest. Corresponding filters may be applied to extract pressure pulses originating from breathing or other physiological signals, which may be used separately or in combination with the heart pulse rates to monitor the fluid flows in the cardiovascular system.

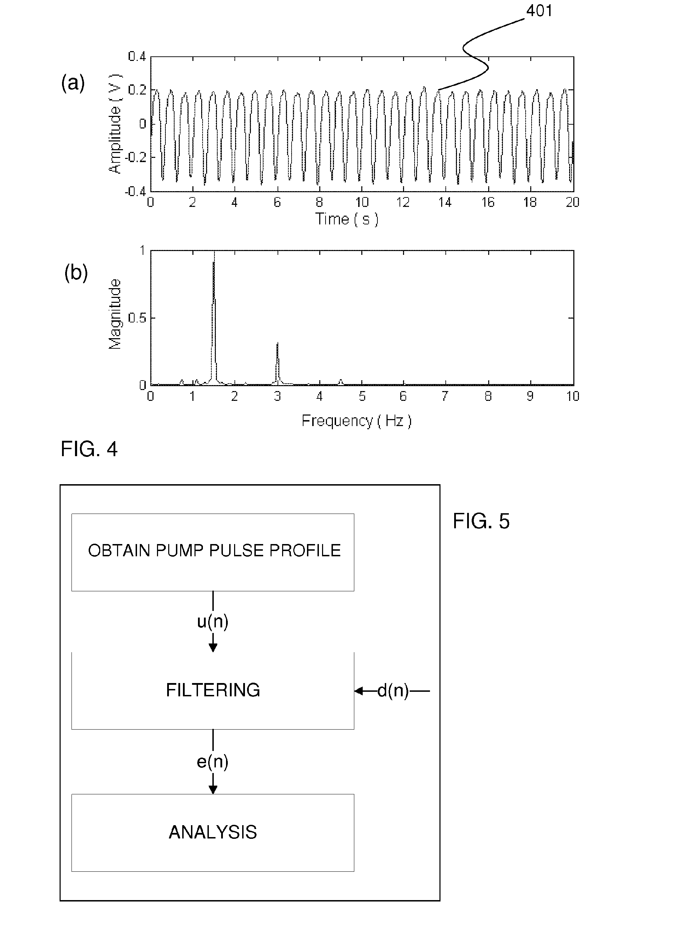

After the pre-processing in the data acquisition part 28, the pre-processed pressure signal is provided as input to a main data processing part 29, which executes the inventive data processing. Fig. 4(a) shows an example of such a pre-processed pressure signal 401 in the time domain, and Fig. 4(b) shows the corresponding power spectrum, i.e. the pre-processed pressure signal in the frequency domain. The power spectrum reveals that the detected pressure signal contains a number of different frequency components emanating from the blood pump 3. In the illustrated example, there is a frequency component at the base frequency (f₀) of the blood pump (at 1.5 Hz in this example), as well as its harmonics 2f₀, 3f₀ and 4f₀. The base frequency, also denoted pump frequency in the following, is the frequency of the pump strokes that generate pressure waves in the extracorporeal circuit 20. For example, in a peristaltic pump of the type shown in Fig. 3, two pump strokes are generated for each full revolution of the rotor 3a. Fig. 4(b) also indicates the presence of a frequency component at half the pump frequency (0.5f₀) and harmonics thereof, in this example at least f₀, 1.5f₀, 2f₀ and 2.5f₀. Fig. 4(b) also shows a heart signal (at 1.1 Hz) which in this example is approximately 40 times weaker than the blood pump signal at the base frequency f₀.

Typically, the surveillance device 25 is configured to continuously process the time-dependent pressure signal(s) to isolate subject pulses originating from for instance a patient's heart. This processing is schematically depicted in the flow chart of Fig. 5. The illustrated processing involves a step 501 of obtaining a pump pulse profile u(n) which is a predicted temporal signal profile of the pump pulse(s), and a step 502 of filtering the pressure signal d(n), or a pre-processed version thereof, in the time-domain, using the pump pulse profile u(n), to essentially eliminate or cancel the pump pulse(s) while retaining the subject pulse(s) contained in d(n). In the context of the present disclosure, n indicates a sample number and is thus equivalent to a (relative) time point in a time-dependent signal. In step 503, the resulting filtered signal e(n) is then analysed for the purpose of monitoring the aforesaid parameter.

The pump pulse profile is a shape template or standard signal profile, typically given as a time-sequence of data values, which reflects the shape of the first pulse in the time domain. The pump pulse profile is also denoted "predicted signal profile" in the following description.

By "essentially eliminating" is meant that the pump pulse(s) is(are) removed from the pressure signal to such an extent that the subject pulse(s) can be detected and analysed for the purpose of monitoring the aforesaid parameter.

By filtering the pressure signal in the time-domain, using the pump pulse profile, it is possible to essentially eliminate the pump pulses and still retain the subject pulses, even if the pump and subject pulses overlap or nearly overlap in the frequency domain. Such a frequency overlap is not unlikely, e.g. if one or both of the pump and subject pulses is made up of a combination of frequencies or frequency ranges.

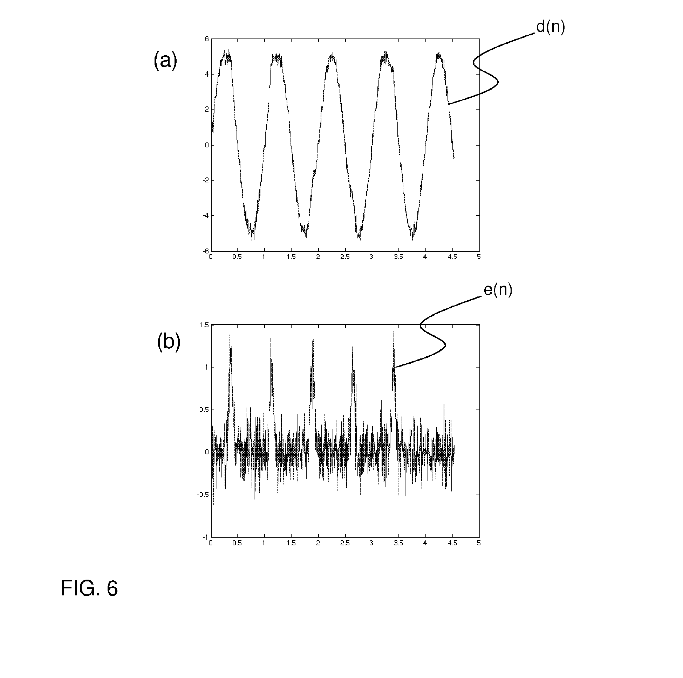

The effectiveness of the inventive filtering is exemplified in Fig. 6, in which Fig. 6(a) shows an example of a time-dependent pressure signal d(n) containing pump and subject pulses with a relative magnitude of 10:1. The pump and subject pulses have a frequency of 1 Hz and 1.33 Hz, respectively. Due to the difference in magnitude, the pressure signal is dominated by the pump pulses. Fig. 6(b) shows the time-dependent filtered signal e(n) that is obtained after applying the inventive filtering technique to the pressure signal d(n). The filtered signal e(n) is made up of subject pulses and noise.

The main data processing part 29 executes the aforesaid steps 501-503 of Fig. 5. In step 502, the main data processing part 29 operates to filter the pre-processed pressure signal in the time domain, and outputs a filtered signal or monitoring signal (*e*(*n*) in Fig. 5) in which the signal components of the blood pump 3 have been removed. The monitoring signal still contains any signal components that originate from the subject (cf. Fig. 6(b)), such as pressure pulses caused by the beating of the patient's heart, breathing or other physiological signals, the latter two which, in a case where the heart is the primary source for analysis, also may be removed to reduce noise from the heart pulses to facilitate the analysis. In another situation breathing is the primary source for analysis, and the heart and other physiological signals may be removed. The signal components may additionally involve artificial origin accounting for modulation from unwanted noise, for instance by a separate, external pressure inducing component, such as integrated in a blood pressure cuff, or the blood pressure cuff itself with pressure waves induced by puffing air into the cuff. A further source of unwanted noise may origin from vibrations, and thus pressure waves, resulting from coughing, sneezing, vomiting and seizures.

Hence, depending on implementation, the surveillance device 25 may be configured to apply filtering to the monitoring signal to isolate signal components originating from a single cyclic phenomenon in the subject, such as the heart pulse or breathing. Alternatively, such signal component filtering is done during the pre-processing of the pressure signal (by the data acquisition part 28). The signal component filtering may be done in the frequency domain, e.g. by applying a cut-off or band pass filter, since the signal components of the different cyclic phenomena in the patient are typically separated in the frequency domain. Generally, the heart frequency is about 0.5-4 Hz, the breathing frequency is about 0.15-0.4 Hz, the frequency of the autonomous system for regulation of blood pressure is about 0.04-0.14 Hz, the frequency of the autonomous system for regulation of body temperature is about 0.04 Hz.

The surveillance device 25 may be configured to collect and store data on the evolution of the amplitude, phase, shape, etc, e.g. for subsequent analysis in connection with monitoring the condition of the subject.

The surveillance device 25 may be configured to monitor the fluid flows in the cardiovascular system of the subject, in particular cardiac output (CO) and access flow Qₐ. This may be done by monitoring characteristics of a signal component originating from, e.g., the patient's heart or breathing system in the monitoring signal or the monitoring signal itself where the composite signal is analysed.

The extracorporeal circuit 20 may have the option to operate in a hemodiafiltration mode (HDF mode), in which the control unit 23 activates a second pumping device (HDF pump, not shown) to supply an infusion solution into the blood line upstream and/or downstream of the dialyser 6, e.g. into one or more of tube segments 2, 5, 10 or 12.

In addition, an external signal source, not shown, such as a photoplethysmograph (PPG) or an electrocardiograph (ECG) signal may be used as a timing reference to the pressure based signal originating from the actuation of the heart.

### III. EXAMPLE OF A FISTULA

For a better understanding of the inventive concept, for instance with regards to measuring flows in the access system, which is related to a fistula, the anatomy and interconnection with a fistula will be described in the following with reference to Fig. 2.

Fig. 2 discloses a forearm 200 of a subject. The forearm 200 comprises an artery 201, in this case the radial artery, and a vein 202, in this case the cephalic vein. The blood flow in a fistula is referred to as access flow Qa, and in Fig. 2 the blood flow in the artery (201) and vein (202) are indicated with arrows. Openings are surgically created in the artery 201 and the vein 202 and the anastomosis are connected to form an anastomosis 203, in which the arterial blood flow is short-circuited to the vein. Such a configuration with the anastomosis 203 and nearby sections of the artery 201 and vein 202 are commonly referred to as a fistula 208. Due to the fistula, the blood flow through the artery and vein is increased and the vein forms a thickened area downstream of the connecting openings. When the fistula has matured a few months after surgery, the vein is thicker and may be punctured repeatedly.

An arterial or withdrawal device 211 in the form of a needle 204, to which is connected a piece of arterial or withdrawal tube 205, is placed in an upstream position 209 in the fistula, in the enlarged vein close to the connected openings and a venous or return device 212 also in the form of a needle 206, to which is connected a piece of venous or return tube 207, is placed in a position downstream 210 of the arterial or withdrawal needle 204, normally at least five centimetres downstream thereof. The withdrawal 205 and return 207 tubes are connected to an extracorporeal circuit (not shown) such as described in Fig. 3. In use, the withdrawal tube 205 may transport blood from the artery 201 via the arterial or withdrawal needle 204 to an inlet of the extracorporeal circuit, and the return tube 207 then returns the treated blood from an outlet of the extracorporeal circuit to the vein 202 via the venous or return needle 206. Arrows at the ends of the blood lines (205, 207) indicate the direction of blood flow in a normal configuration.

The vascular access may also be an arterio-venous graft, Scribner-shunt, one or more catheters, a double lumen catheter or other similar arrangements. For the purpose of the following discussion, the blood vessel access is assumed to be a fistula. The withdrawal and return needles may also be catheters. The withdrawal and return devices generally comprises a needle or catheter, a tubing and a connector (not shown) connecting the tubing to the needle or catheter.

The needles 204 and 206 are connected to a tube system, shown in Fig. 3, forming an extracorporeal blood flow circuit 20 of the type which is used for dialysis.

### IV. MODELLING OF A CARDIOVASCULAR SYSTEM

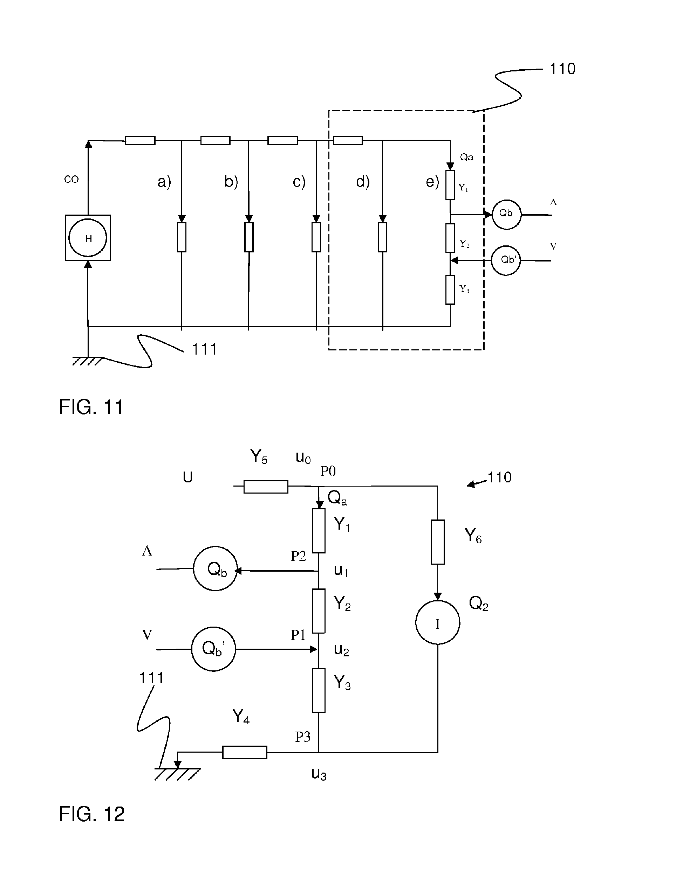

For a better understanding of the inventive concept, a hydraulic model of the cardio-vascular system will be described in the following with reference to Fig. 11.

The access flow Qa in a fistula is related to other flows in the cardiovascular system, such as Cardiac Output (CO), which is the total blood flow from the heart.

Generally, fluid flow rate (Q) and flow admittance (Y) are measures which may be used to describe the function and condition of a cardiovascular system. Particularly, the fluid flow rate (Q) and flow admittance (Y) may provide vital information of the heart function and condition of a fistula, and how it changes over time, for instance during a treatment or between treatments. Fluid flow rate (Q) may also be used to determine where, for instance in a fistula, a problem may reside. The inverse of admittance (Y) is impedance (Z), both which may be referred to in the following.

Fig. 11 shows a schematic diagram of a hydraulic model of the cardio-vascular system connected to an extra-corporeal circuit. The blood flow at any location in the cardio-vascular system is related both to the activity of the heart (H), e.g. referred to as the Cardiac Output (CO), and to the distribution of vascular flow impedance (Z) in the vascular system. The vessels actively change diameter under the influence of physiology, for instance by temperature regulation, or therapy, vasoconstrictors decrease vessel diameter and increase impedance, while vasodilators increase vessel diameter and decrease impedance due to heart activity regulation by the autonomous system. Hence, an increase in impedance decreases the Cardiac Output (CO). Due to the cardiac output (CO) and admittance (Y) in the cardio-vascular system, the mean arterial pressure (MAP), is generated. The Cardiac Output (CO) is distributed into the organs and limbs through a complex network of blood vessels, illustrated with branches a) - e) in Fig. 11. Each branch is associated with a certain flow admittance.

As long as the impedance properties of the vascular system are fairly stable over an investigated period of time, the relative flow variation at a certain point may be approximated by the relative flow variation at any other points of the system according to changes in the activity of the heart. Although not necessarily being linear, the relationship between a change of flow in one branch to the corresponding value of another branch is generally largely governed by a certain mathematical model of the cardiovascular system. Therefore, access flow variations may for instance be determined from Cardiac Output (CO) variations and vice versa. Variations of blood flow to e.g. an organ or a limb may be estimated based on determinations of blood flow variations in another location. Absolute flows however, need to be determined via calibration with respect to reference measurements or hydraulic models of the vascular system. Also the model may be subjected to calibration, e.g. by any of the previously mentioned reference methods. At least one point is needed for the calibration, but additional points would allow determination of linear and non-linear relationships that may exist between the measured pressure and the blood flow. For instance, provided that aforesaid relationship is linear, two reference measurements would suffice to provide calibrated fluid flow rate values of the method according to the invention.

A blood access is exemplary modelled with three admittance symbols to the right in the figure within the dashed rectangle 110, which is enlarged and explained in detail with reference to Fig. 12. Here, the access flow is represented by Qa, Qb is blood flow withdrawn from the blood access and Qb' is the blood flow returned to the blood access during dialysis. "A" and "V" represents the "arterial" and "venous" side of the extra-corporeal circuit. Also shown in the model are body admittance boxes Y₁, Y₂ and Y₃ related to the flow and pressure characteristics of the vascular branch comprising the fistula. The earthing symbol 111 indicates that the absolute pressure is low at the venous return to the heart, i.e. close to zero relative the atmospheric pressure according to known principles.

Fig. 12 shows a close-up 110 of a blood access branch of the cardio-vascular system of Fig. 11, the branch also comprising a fistula here represented by the three flow admittances Y₁, Y₂ and Y₃. Other fistula configurations may be modelled according to the same principles.

The fistula configuration has been translated into a hydraulic model as is shown in Fig. 12, where the blood flow in the access devices is indicated with arrows. Table 1 below lists the modelling parameter definitions that are used in Fig. 11 and Fig. 12 and related to the analysis. Additionally, P0 denotes a position in the artery close to the anastomosis. P1 denotes an arterial acccess point in fistula, P2 denotes a venous access point in fistula, and P3 denotes a common venous return point for the blood flow in the artery, i.e. Qₐ + Q₂.

Since the model takes dynamic as well as static effects into account, it may be applied to pressure levels as well as pressure variations, hence including effects due to propagation of pressure pulses and admittance in the system. In dynamic modelling, frequency dependence arises due to damping in the system. Generally, the pressure pulse propagation speed may be modulated by e.g. blood pressure.

**Table 1. Modelling parameter definitions**

| |
|---|
| UFR: ultrafiltration rate |
| Q_{b}: blood flow of dialysis machine |
| Z=impedance |
| Y=1/Z, i.e. admittance is the inverse of impedance |
| |
| Y₁: flow admittance between anastomosis (P0) and arterial access point (P1), 'pre-fistula admittance' |
| Y₂: flow admittance between arterial (P1) and venous (P2) access points, 'intra-fistula admittance' |
| Y₃: flow admittance between venous (P2) access point and common venous return vessel (P3), 'post-fistula admittance' |
| Y₄: flow admittance in vein between common venous return vessel (P3) and venous return to heart (111) |
| Y₅: flow admittance in artery entering the fistula |
| Y₆: flow admittance of vessels supplying tissue in the arm/hand access |
| |
| u₀: arterial blood pressure at the anastomosis (P0), e.g. reflected by mean arterial pressure (MAP) |
| Normal position of access needles: |
| u1: blood pressure at the arterial access point 1 (P1) |
| u2: blood pressure at the venous access point 2 (P2) |
| u3: blood pressure at common venous return vessel (P3) |
| |
| Q_{b}:blood flow pumped out of the fistula |
| Q_{b}': blood flow pumped back to the fistula: Q_{b}-UFR |
| Q₂: arterial blood flow required by the tissues downstream of the fistula, |
| Qₐ: blood access flow entering the fistula |

In the model, it has been assumed that the blood pressure of the artery e.g. reflected by mean arterial pressure MAP is controlled by autonomous system to a constant value (u₀), the demand of nutrition and oxygen of tissue, the arterial blood frlow in the branch of the fistula is constant (Q₂) and that all flow admittances (Y₁ - Y₄) do not vary. Both Y₅ and Y₆ are omitted in the modelling, since they are assumed to be very large.

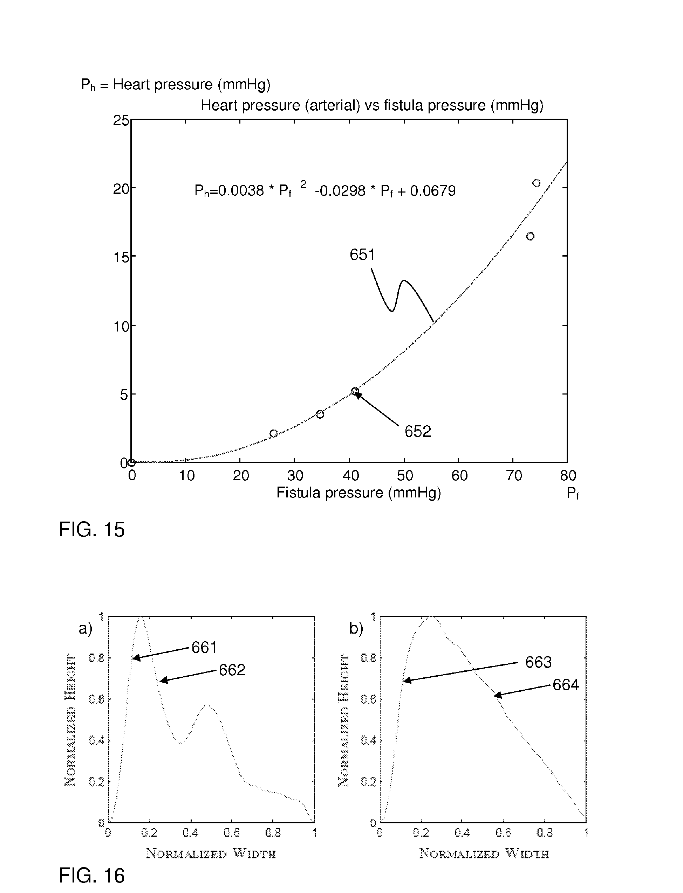

Fig. 15 shows how the amplitudes of the heart pressure signals at A or V in Fig. 12 depend on the fistula pressure, e.g. it is possible to determine a relationship from the heart amplitude in A or V with the fistula pressure for the corresponding access point in the fistula.

### V. CARDIOVASCULAR FLUID FLOW RATE FROM PRESSURE SIGNAL

In the present invention, methods for analysing the pulse pressure are used to determine a waveform and using this information to calculate cardio-vascular measures such as related to the cardiac performance and blood access branches of the cardiovascular system. The inventive concept is based upon the relation that pressure variations in an extracorporeal circuit in fluid connection with a cardiovascular system may be used to derive pressure and flow variations in the cardiovascular system. Thus, variations in Cardiac Output (CO) and access flow are both related to variations in the heart signal measured by the present invention. Hence, by monitoring pressure variations in the extracorporeal circuit and relating these variations with relevant cardiovascular relationships, cardiovascular fluid flow rates may be determined.

The present invention generally relates to variations in fluid flow rates in a cardiovascular system causing variations in fistula pressure which in turn cause changes in the heart pulses obtained from an extracorporeal pressure sensor, which changes may be quantified by a parameter P involving for instance amplitude, shape, phase and/or frequency or timing.

Monitoring of the cardiovascular fluid flow rates may provide vital information of a subject, for instance cardiac function and/or blood access function, which will be described in more detail in separate sections below.

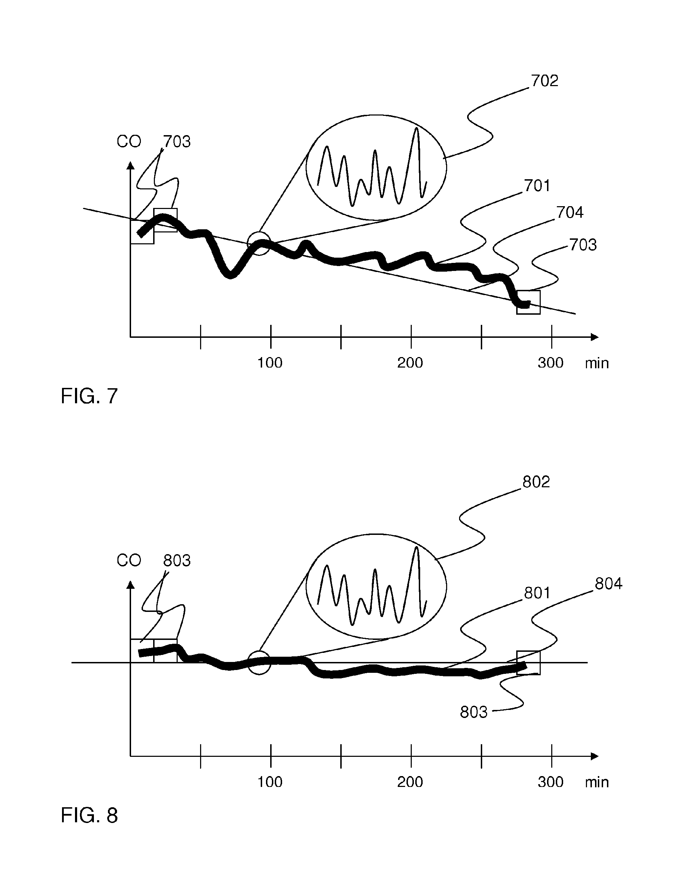

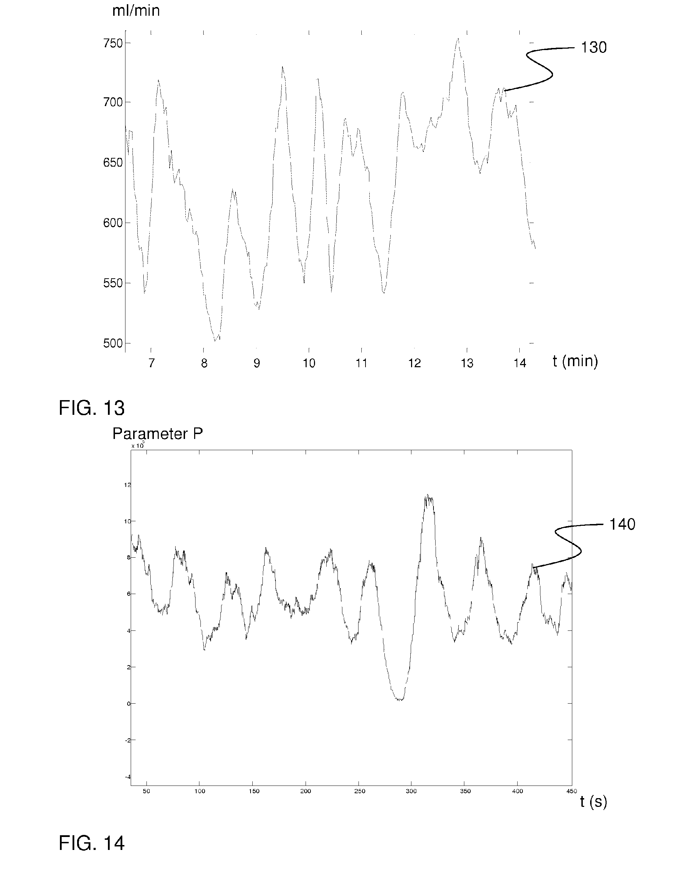

Fig. 7 and Fig. 8 illustrate monitoring of cardiac output variations during two different treatments having durations of approximately five hours each. Similar variations would be seen also in for instance access flow measurements. Similar recordings may be generated for a number of subsequent treatments to get an overall clinical picture. Fig. 7 illustrates measurements from a subject where cardiac output 701 is decreasing during a dialysis treatment, as indicated by trend line 704. Fig. 8 illustrates measurements from a patient where cardiac output 801 is relatively stable, as indicated by trend line 804. In the present invention, it has surprisingly been found that periodic variations can been seen on a smaller time scale. The close-ups 702 and 802 in the figures illustrate segments where such periodic variations in the range of approximately 50 seconds and representing a relative flow variation of about 10-30% may be seen. These periodic variations are explained in more detail in connection with Fig. 13 and Fig. 14 below. The squares 703 and 803 illustrate segments of time periods wherein calibrations have been performed with a reference method during the treatment. Calibration may of course be performed anytime during the treatment, and may be used between treatments, for instance by storing the values in a memory, but is preferably at least performed at the beginning and/or end of the treatment. Two initial calibrations are also shown which may provide improved accuracy, for instance to determine and/or improving e.g. a linear relationship between relative measurements and absolute values.

The analysis of pressure variations in the pressure signal may comprise various measures, such as amplitude, shape and phase. For instance, the amplitude of peak maxima in the pressure signal may be proportional to a cardio-vascular flow. Additionally, due to damping and delay of the frequency components of the heart pulses affecting their shape and/or phase, predetermined profiles representing a particular cardio-vascular flow may be mapped against a measured pressure pulse profile.

By way of example, plots representing access flow rate variations (on the Y-axis) obtained with a prior art method, and with the present invention will now be illustrated in Fig. 13 and Fig. 14, respectively. The horizontal axis represents different time periods of approximately six to 8 minutes each. In both figures, variations are present, the variations having a period in the range of 40 to 60 seconds.

Fig. 13 and Fig. 14 show variations in access flow rate in a relatively short time-scale as indicated with the close-ups 702 and 802 in Fig. 7 and 8 respectively. Fig. 13 and Fig. 14 do not necessarily correspond to the same time period of the same treatment. Fig. 13 shows actual access flow variations 130 obtained with an ultrasound (Doppler) measurement. The x-axis represents time in minutes and the y-axis represents flow rate in ml/min. Short-term variations in the access flow rate of approximately a minute in duration are clearly visible.

Fig. 14 shows a cross-correlation measure 140 between heart signals obtained from the arterial and venous pressure sensors according to one aspect of the present invention. Alternatively, either of the heart signals from the arterial or venous pressure sensors may be used, although a cross-correlation between the two may suppress unwanted components. The cross-correlation measure is shown before calibration and indicates the relative variation. The x-axis represents time in seconds and the y-axis represents access flow rate of an arbitrary unit calculated from the amplitude of the heart pulses. Other parameters that may be used in the calculation include shape, frequency, phase and variability. Also, amplitude variations 140 of similar periodicity are clearly visible. Hence, Fig. 14 illustrates that access flow rate variations, similar to those in Fig. 13, may be monitored by pressure sensors in the extra-corporeal circuit. These variations may be used to better determine the absolute value of both cardiac output and access flow when the corresponding reference methods are used for calibration.

An advantage with the present invention is that it may be built into for instance a dialysis monitor and that it does not require additional instruments and sensors, particularly when the monitor is further configured to measure reference values of for instance access flow Qa and Cardiac Output (CO). Especially, it provides a continuous "beat-to-beat" measure. Actually, even different measurements within a pulse is possible with the present invention.

There are of course other techniques for calculating the evaluation parameter value P, including other types of time domain analyses, as well as different types of frequency domain analyses.

Other factors, such as the medical history of the patient, e.g. heart status, blood pressure and heart rate may also be utilized for improving the vascular flow determination.

Pressure data extracted from the measurement signal may be represented as a temporal pulse profile in the time domain. The temporal pulse profile may be transformed into a frequency spectrum and/or a phase spectrum as another pressure data representation. From the pressure data, a parameter value may be calculated. For example, the parameter value may be related to the amplitude, shape, frequency and/or phase, or timing of the pressure pulse.

The performance of the reference methods may be improved since the measurement period of these methods are shorter in time than the period of the variations presented in Fig. 13 and Fig. 14. Thus, when an absolute value is obtained by the reference methods it will be affected by the magnitude of the inherent flow variations and it will also be dependent on when in time the measurement is performed. The variation in Fig. 14 may be used in order to improve performance of the calibration methods by taking into account e.g. the time of injection, duration of injection, and circulation times in the cardio-vascular system.

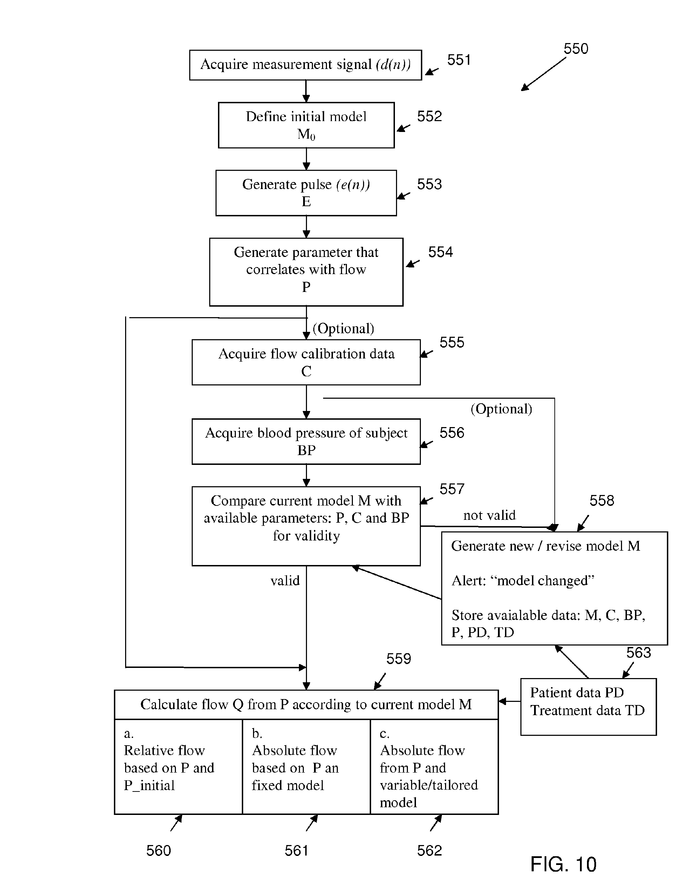

With the pulse wave analysis of the present invention, it is now possible to improve the absolute measurements. It is even possible to improve the reference measurement methods. The high resolution in the pulse wave analysis reveals more details of the relative variations of access flow and cardiac output than previously perceived. Further details are given below in connection with calibration step 555 of Fig. 10.

According to one way of improving absolute measurements of average fluid flow rate, a reference fluid flow rate (Qref) is calculated at a time point when the calculated relative fluid flow rate (Q) corresponds to an average fluid flow rate. In this way, reference measurements are determined at instances of average fluid flow rates, suppressing effects from variability in the fluid flow rate. Thus, it is not necessary to calculate a relationship between reference values relative measurement values.

Additionally, provided that the properties of the vascular system remain constant over time, i.e. no stenosis formation, calibration of the cardiac output measurement according to the present invention may remain valid and be used for monitoring of long-term changes of the cardiac output.

The present invention discloses a method for measuring the access flow rate, cardiac output and other cardiovascular flow rates almost continuously, dependent essentially only on update frequency and subject pulse rate. The variability associated with reference methods due to cardiovascular flow rate variation may thus be reduced by using data for the corresponding time period together with an estimation of the variability obtained by the present invention.

### VI. MONITORING CARDIAC OUTPUT

Cardiac output is the quantity of blood pumped each minute by the heart into the aorta, i.e. the total blood flow in the circulation of a subject. During a dialysis treatment, the cardiac output is often referred to as the intra-dialytic cardiac output.

According to one embodiment of the present invention, variations in Cardiac Output (CO) are obtained by tracking the amplitude in the heart pulses obtained from an extracorporeal pressure sensor, based on the relation that a variation in Cardiac Output (CO) causes a variation in the amplitude of the heart pulses obtained from an extracorporeal pressure sensor. As an alternative to tracking the amplitude, also shape, phase and frequency or the integral of values within a time window may be used. The time window may contain one or several heart pulses as well as fractions of heart pulses. Alternatively, the sum of absolute or squared values within a time window may also be used, i.e. an energy equivalent.

Calibration of the relative measure to get an absolute measure of intra-dialytic cardiac output (CO) may be obtained by using an indicator dilution method as is described in international patent application published as WO 2005/049113, in which concentrated saline is used as the indicator and the conductivity in the spent dialysate is measured. A known amount of concentrated NaCl, e.g. 2 ml, in the form of a short duration bolus is given in the venous return line of the extracorporeal circuit. On its way back to the subject's heart, the bolus will meet returning blood from the rest of the body, pass the lungs and go back to the heart. It will then be pumped out to the body again with a flow rate determined by the Cardiac Output. As the bolus spreads through the cardio-vascular system and out in the extracorporeal circuit, a fraction of the original bolus of NaCl will be measured. The fraction reaching the spent dialysate will be clearance (K), which is the flow of blood passing the dialyzer that is completely cleared from a waste substance such as urea, divided by Cardiac Output, i.e. (K/CO). This fraction is measured as a conductivity increase in the spent dialysate, and the area under the curve is measured. The conductivity area is converted to a NaCl concentration area using the known specific conductivity of NaCl. Multiplication with the dialysate flow rate then gives the total amount of NaCl in the spent dialysate. The fraction of this measured outlet pulse in relation to the original bolus will then equal K/CO, so that if clearance (K) is measured, the Cardiac Output (CO) may be calculated.

Alternatively, measurements of the bolus dilution may also be performed with ultrasound detection, relying on measurement of transient changes in ultrasound velocity induced by an indicator e.g. saline that has been added to the blood. A known amount of an indicator substance e.g. saline, is injected into the blood stream and its dispersion cause changes in the ultrasound velocity which is related to the concentration of the indicator.

Additionally, with embodiments of the present invention it is possible to improve the above mentioned reference method and other reference methods. A better estimate of an average absolute value from a reference measurement may be accomplished by accounting for the variations in the access Cardiac Output (CO) measured by continuous measurements according to embodiments of the present invention. For instance, by continuously tracking a relevant parameter of the blood pressure pulses, it is possible from the observed variations to determine when a calibration method should begin for instance a bolus injection and when to start detection of a response of the bolus injection. If two or more absolute values are obtained from a calibration method, preferably close in time, at different time instances in the variation curves in Fig. 14, then these absolute values may be combined together with the time instances in the variation curves in order to obtain a better estimate of the absolute average value. For example, if two values are obtained at the time instances of the maximum and minimum values of the variation curves, then a better estimate of the average absolute value would be the mean of these two values. Alternatively, the middle point in time between a max and min may be used for reference measurement to get a good estimate of the average flow.

### VII. MONITORING ACCESS FLOW

The branch of the cardio-vascular system that passes a blood access is associated with an access flow (Qa), which may be determined with the present invention.

According to embodiments of the present invention, variations in the access flow are obtained by tracking the amplitude, shape, frequency and/or phase, or timing information of for instance the heart pulses obtained from an extracorporeal pressure sensor, based on the relation that a variation in access flow causes a variation in for instance the amplitude of the heart pulses obtained from an extracorporeal pressure sensor. Instead of the amplitude, any of the other characteristics may equally well be used. This is illustrated in Fig. 14. As an alternative to tracking the amplitude, shape, phase and frequency or the integral of values of the pulse profile within a time window may also be used. The time window may contain one or several heart pulses as well as fractions of heart pulses. Alternatively, the sum of absolute or squared values within a time window may also be used.

Calibration of the relative measure to get an absolute measure may be obtained with for instance conductivity-based methods, such as referred to as a Cond-Step method, as is described in international patent application published as WO 03/066135, in which a difference in conductivity between two measurements is measured, with the blood flow being reversed between the two measurements, i.e. the connectors of the venous and arterial needle are interchanged causing recirculation of the withdrawn blood flow Qb. This will decrease the treatment efficiency to a certain degree, from which the access flow rate can be calculated. The change in treatment efficiency is measured as a change in the outlet conductivity. The treatment efficiency is proportional to the distance between inlet and outlet conductivities of the dialysis fluid. The access flow rate is inversely proportional to the change in this distance.

When performing this measurement it is necessary to ensure a difference between the inlet and outlet conductivities that is large enough. If there is no difference there will be no conductivity change when the needles are switched, and no calculations can be performed. Therefore any measurement with the Cond-Step method starts with a step in the inlet conductivity. This step is used also to obtain a clearance value, which is also needed in the access flow calculation.

The Cond-Step reference method provides up to approximately four measurement values per hour during a treatment. Each value represents access flow rate during period of approximately ten to twenty seconds and due to variations of the access flow rate with a cycle of about a minute during treatment, this and other reference methods provide only limited approximations of average access flow rate.

Other reference methods involve urea measurements such as described in WO 00/24440 or dilution measurements such as a method of Transonic where magnitude of access recirculation is determined from dilution measured with ultrasound as the ratio between the amount of indicator arriving in the arterial line during its first transit and the injected amount of indicator. Access flow Qa is related to the forced recirculation R of the reversed access position, blood flow Qb and the ultrafiltration flow rate UFR according to : Qa=(1-R)*(Qb-UFR)/R.

Similarly to the Cardiac Output measurements, embodiments of the present invention may be also used to improve the above mentioned and other reference methods for access flow measurements.

### VIII. ANALYSIS

On a general level, the detection may involve calculating an evaluation parameter value based on the isolated pressure data resulting from the aforesaid signal extraction. The evaluation parameter value is then analysed as part of a process for determining flows of the cardiovascular system.

Different techniques for calculating such an evaluation parameter value are further disclosed and exemplified in Applicant's International patent publication WO2009/156174, entitled "Methods and Devices for Monitoring the Integrity of a Fluid Connection".

There are of course other techniques for calculating the evaluation parameter value, including other types of time domain analyses, as well as different types of frequency domain analyses, e.g. as indicated in the following.

Pressure data extracted from the measurement signal may be represented as a temporal pulse profile in the time domain. The temporal pulse profile may be transformed into a frequency spectrum and a phase spectrum, or only a frequency spectrum. From the pressure data, a parameter value may be calculated. The parameter value may be related to the amplitude, shape or timing of the pressure pulse, e.g. timing of a well-defined pulse feature such as zero-crossing or max/min occurences.

Fig. 10 is a flow chart that illustrates an overview 550 of the process of determining fluid flows in a cardiovascular system using one or more pressure sensors in an extracorporeal system according to embodiments of the present invention. It shows an input of a measurement signal in 551 comprising pressure data as measured by one or more pressure sensors. Embodiments of the present invention may advantageously be described in terms of a model describing mathematical relations between flows and pressures of the circulatory system of a subject. It may be a generic model for any subject or individualized for a particular subject. The model may include the relationship between the pressure data extracted from the measurement signal and the corresponding pressure within the fistula. For instance, an initial model M₀ is defined 552 comprising model type and model definition parameters, i.e. model settings. The current model M for flow determination is set to the initial model M₀. Hence, the initial model is assigned to the current model M. The model settings may be predefined, generated based on information about the subject or a definition of the extracorporeal treatment, as is described below in connection with steps 559.

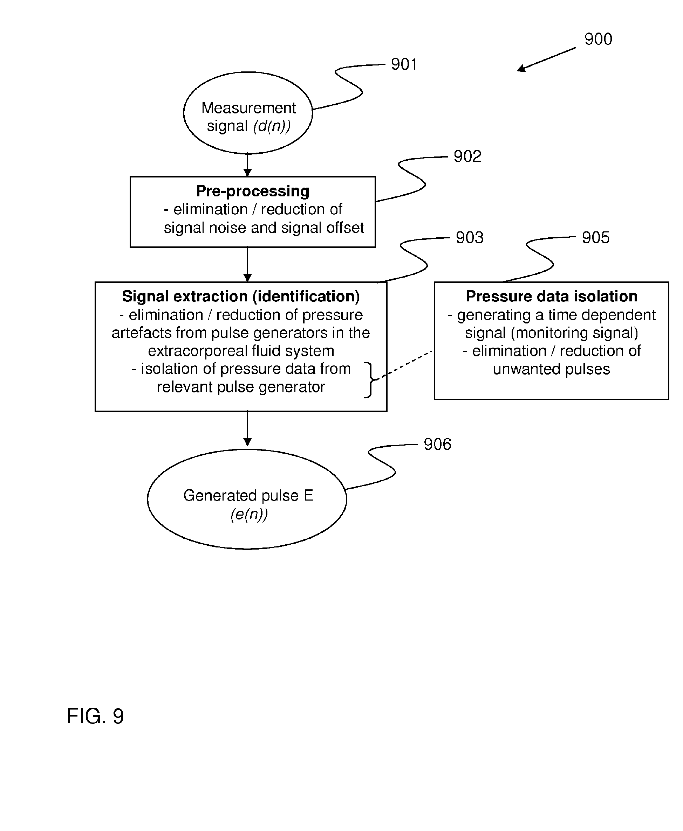

Next, a pulse measure E, i.e. pulse profile *e(n)*, is generated 553 from the pressure measurement signal according to the procedure described in connection with Fig. 9 below. The pulse measure E is a temporal signal profile from which various features can be calculated. Based on E, a parameter P is generated 554, which parameter P relates to a fluid flow rate Q of the cardiovascular system. In the process of generating the parameter P, various features of E may be utilized, alone or in combination, such as amplitude, shape, frequency and/or phase or timing. The generation process may include mathematical operations such as peak-to-peak detection, integration, summation, variation (e.g. standard deviation), polynomial evaluation, etc. It may for instance involve the integral under the curve within a time window, sum of absolute or squared values within a time window or the difference between two pulses in the temporal signal profile. The relationship between the model M, pulse E and parameter P may be expressed as P=P(E), i.e. the parameter is a function of the pulse E, and Q=M(P), i.e. the fluid flow rate is a function of the model in which the parameter P is used, possibly in combination with additional parameters such as calibration C and blood pressure BP.

The parameter P may be an amplitude of the heart pulse. A cardiovascular flow rate may then be derived from linear or non-linear mathematical relationships. In an example of a non-linear relationship, a cardiovascular flow rate may be determined from a first relation between heart pulse amplitude Pₕ and fistula pressure P_{f}, such as the second order polynomial and curve 651 of Fig. 15, together with a second relation between the fistula pressure and access flow in the fistula. This relation and its connection with the model of Fig. 12 is further described in connection with section c) below. Over limited intervals, the non-linearity may further be approximated to a linear dependency. Measurepoints 652 indicate fistula pressure measurements to which the polynomial has been adjusted.

Alternatively, the parameter P may be generated based on shape data. Here, the parameter P may quantify the likeness of the pulse profile *e(n)* E to typical profiles representing high or low fluid flows, which may be illustrated with Fig. 16. Fig. 16 shows the two phases of a heart pulse waveform , i.e. the rising edges 661, 663 and falling edges 662, 664 of the pulse (the anacrotic and catacrotic phases). The heart pulse wave form from a vessel with a normal, moderate fluid flow rate show a "triphasic" shape as seen in section a) of Fig. 16. Higher blood pressure imply higher blood flow when the properties of the vascular system are essentially unchanged, meaning for instance that a pulse shape according to section b) of Fig. 16 represents a higher flow than the pulse shape of section a) of Fig. 16. The heart pulse wave form in a vessel with higher fluid flow rate tends to render the shape more "biphasic", such as seen in section b) of Fig. 16. Note that the parameter P may be based on a plurality of pulse measure E values from e.g. a defined time period.

The fluid flow Q may then be calculated from the parameter P according to the model M as described in connection with step 559 below. Optionally, the fluid flow Q may be calibrated in a calibration step 555, in which flow calibration data C is obtained by performing one or more reference measurements of the flow. Depending on the model used one, two or more reference measurement may be required. A first order model requires one reference measurement if either the slope or the offset is known, two reference measurements are required if both slope and offset are unknown. Higher order models require additional calibration data. The calibration may be performed during the same or during different extracorporeal treatments for the same subject or possibly for a group of subjects.

For instance, in embodiments of the present invention, it is possible to obtain greater accuracy in absolute average values from reference measurements since we now know the timing of a particular reference measurement with respect to the flow determination of the present invention. Previously, when performing calibration, it has been assumed that the measurements have been on the curve of e.g. Fig. 7 or 8. For instance, it was not known if a reference measurement was obtained in a crest or a trough of the short time-scale variations. However, with the new knowledge of the short-time variations, it is seen that reference measurements are dependent on when in the variations measurements are obtained. Due to these variations, conventional reference methods based on isolated measurements depend on when measurements are performed. We now know when in short time-scale variations a particular reference measurement has been obtained, providing information as to how reference values relate to measurements on a longer time-scale. Embodiments of the present invention thus enable obtaining average values with less variability which may be used to improve the reference methods for obtaining relations to absolute values. The improvements relating to reference measurements benefit from particularly two aspects when having knowledge about the timing information. Firstly, the time of occurrence of reference measurements may be related to the occurrence of the relative measurements for a more precise determination of reference measurements. Secondly, the timing of reference measurements may be controlled with timing from the relative measurements according to the present invention. Since cardiovascular flow is affected by the blood pressure in the cardiovascular system, it may be relevant to include also blood pressure BP in the model to account for this effect, and thus improve the model. Blood pressure measurement may be performed in step 556. The measurement may obtain the Systolic, Diastolic, Mean Arterial Pressure MAP or a regional blood pressure of the subject. The then obtained current parameter P, calibration data C, blood pressure BP and previously recorded data such as information on the patient PD and the treatment TD may then be compared 557 with the current model M to check if the model is still valid for calculation of the fluid flow Q. The patient specific data PD and treatment specific data TD may have been recorded during the same treatment session or one or more previous treatment sessions.

If a satisfactory fit of the parameters C, P, BP, PD and TD with the model is achieved, the fluid flow Q is calculated in accordance with step 559. However, if a satisfactory fit cannot be achieved or combinations of the values of P, C, BP, PD and TD yield unlikely results with the model, the model is deemed "not valid" and the model is revised 558. Hence, a new or revised model is generated based on the same parameters C, P, BP, PD and TD. Optionally, new calibration measurements may be obtained. Also optionally, an alert "new model was required" may be issued and displayed. In case such an alert is given several times in sequence, a "not possible to adapt model"-message may be given to inform the operator that the flow measurement procedure may not be reliable. Alternatively, as a safety action, the measurement procedure may be aborted. Measurement readings, calibration data and the new model definition parameters may also be stored for subsequent use.

In fluid flow calculation step 559, the fluid flow Q is calculated based at least on the parameter P according to current model M. Other patient or treatment specific data (PD or TD) may also be provided 563 as input in the calculations. Three variations of models which may be used will now be briefly explained:
a) The model may be based upon a relative vascular flow measure R which is calculated based at least on the current parameter P and a normalization value Pi (P_initial), which for instance may be an initial fluid flow parameter value acquired at the beginning of a treatment. The relative flow measure R may be calculated as a ratio directly (P/Pi) or after a linear or non-linear transformation of P and Pi expressed as R=Q/Qi=M(P,Pi). Relative changes would be detected, such as stable or decreasing cardiac output as illustrated in Fig. 8 and Fig. 7 respectively.
b) The model may be based upon a fixed model of the absolute fluid flow Q versus the parameter P, i.e. Q=M_{fix}(P). Such a fixed model may have been obtained from assessment and/or comparison with measurement data recorded in previous treatment sessions of the same subject or a group of subjects. The model may be defined as any mathematical function, e.g, linear, non-linear etc, which produce Q based on at least the parameter P. The model may be fixed in the sense that it is unaltered between treatments. Determination of an absolute reference of the fluid flow Q may have been obtained in a previous treatment session.
c) The model may be a variable, tailored model which may be used for calculation of fluid flow Q based on at least the parameter P. The model may involve a plurality of model definition parameters which may be changed in real-time during a treatment and depending on individual and variable characteristics of the subject PD and changes of the treatment condition TD. The model may be linear such that the fluid flow may be calculated as Q = k * P + 1, where k and 1 may be empirically determined from calibration data C. In another variant, the model may be given as a look-up table for different combinations of the parameter P and possibly other parameters such as blood pressure measurement BP, patient information data PD or treatment information data TD. For this purpose, it may be advantageous to obtain continuous blood pressure measurements, such as by using pressure wave velocity of arterial vessels which is disclosed and exemplified in the Applicant's provisional US patent application No. 61/290308 filed 28 December 2009 entitled "MONITORING BLOOD PRESSURE" which was filed concurrently with the present application. According to another variant of the model, it may be given based on Fig. 12. Here, e.g. fluid flow Qₐ = Y₁ (u₀ - u₁), where u₀ is the artery pressure, u₁ is the fistula pressure and Y₁ is the flow admittance between point "0" and "1" in the diagram. Y₁ may be determined if a reference measurement of the fluid flow Q_{ar,} , e.g. with Doppler measurements or Cond-step methods, the fistula pressure u₁, and the artery pressure u₀ are known. Fig. 15 shows that the fistula pressure u₁, e.g. P_{f}, may be modelled based on the heart pulse amplitude measured in the extracorporeal circuit. Alternatively, also heart signal amplitude in the fistula may be derived and used as input for a model for determining the vascular flow. It is therefore possible to calculate the access flow Qa if the artery pressure value u₀ is given since both Y₁ and u₁ are known. Artery pressure uₒ may be obtained by blood pressure measurement. For this purpose, continuous blood pressure measurements may e.g. be achieved by combining an absolute blood pressure determination with continuous relative blood pressure measurements based on determination of pressure wave velocity of arterial vessels.

Fig. 9 is a flow chart that illustrates steps of a signal analysis process 900 according to an embodiment of the present invention. The process 900 operates on measurement data obtained (sampled) from, e.g., the venous, arterial and/or system pressure sensors, thereby generating signal values of a measurement signal comprising a number of pressure induced signal components.

The measurement signal comprises signals originating from one or more sources and thus constitutes a composite signal of the signals from said sources. The measurement signal may be used without further processing, although preferably, the measurement signal is processed for extraction of pressure data originating from a subject pulse generator in the cardiovascular system. The extraction may be performed by filtering to remove unwanted pressure data, such as from a blood pump in an extracorporeal system.

In the vascular system, a subject pulse generator may be a physiological phenomenon, such as the pulsations of from the heart or breathing from the lungs. The signal analysis process may be divided into a pre-processing part 902, a signal extraction part 903 and an analysis part 904. The pre-processing part 902 includes elimination or reduction of signal noise, e.g. measurement noise, and signal offset, e.g. as detailed in the section above relating to the data acquisition part 28. The signal extraction part 903 involves elimination or reduction of pressure artefacts originating from pulse generators in the extracorporeal fluid circuit or in the cardiovascular system and isolation of pressure data originating from the relevant physiological phenomenon. In the context of the present disclosure, "pressure data isolation" 905 denotes a process of generating a time-dependent signal profile of the subject pulse *(e(n))* which is free or substantially free from pressure modulations caused by any unwanted physiological phenomena. Such unwanted physiological phenomena may vary between different applications, and may include breathing, coughing, etc. The elimination of signal noise and signal offset, as well as the elimination of pressure artefacts, may be included in algorithms for pressure data isolation. For instance, the measurement signal may be band pass filtered or low pass filtered to isolate a heart signal, in a way such that signal noise and/or signal offset and/or pressure artefacts are eliminated from the measurement signal. The elimination of pressure artefacts may thus be performed before, after or during the pressure data isolation.

The generated pulse measure E *(e(n))* is input in step 553 in Fig. 10 to generate a parameter P, as further described in connection with Fig. 10.

The calculation may be designed such that the parameter value represents timing, amplitude or shape of the pulse. However, the detection may also be performed in the frequency domain by analysis of the amplitude and/or phase spectrum.

In the simplest case of pressure signal analysis, no pump or other source of pressure artefacts is present in the extracorporeal fluid circuit connected to the subject during the data acquisition. For instance, the pump may have been shut down.

In the general case, however, one or more pumps are running or other sources of cyclic or non-cyclic repetitive and non-repetitive artefacts are present during the data acquisition. Information on the cyclic disturbances may be known from external sources, e.g. other sensors, or may be estimated or reconstructed from system parameters.

Cyclic pressure artefacts may originate from operating a peristaltic pump, repetitive actuation of valves, movements of membranes in balancing chambers. According to the findings in connection with the present invention, artefacts may also originate from mechanical resonance of system components such as swinging movements of blood line energized by e.g. a pump. Frequencies of blood line movements are given by the tube lengths and harmonics thereof and by the beating between any frequencies involved, i.e. between different self-oscillations and pump frequencies. These frequencies may differ between the venous and arterial lines. Mechanical fixation of the blood lines and other free components may remedy the problem of mechanical resonance. Alternatively, an operator may be instructed to touch or jolt the blood lines to identify natural frequencies associated with the blood lines, which information may be used in the analysis for improved removal of components not belonging to the pressure data of interest.

Examples of non-cyclic artefacts are subject movement, valve actuation, movements of tubings etc.

Various techniques for signal extraction will be discussed in a section further below.

The invention has mainly been described above with reference to a few embodiments. However, as is readily appreciated by a person skilled in the art, other embodiments than the ones disclosed above are equally possible within the scope of the invention, which is defined and limited only be the appended patent claims.

For example, the illustrated embodiments are applicable for cardiovascular flow measurements involving all types of extracorporeal blood flow circuits in which blood is taken from a patient's circulation. Such blood flow circuits include hemodialysis, hemofiltration, hemodiafiltration, plasmapheresis, aphaeresis, extracorporeal membrane oxygenation, assisted blood circulation, and extracorporeal liver support/dialysis, bloodbanking, blood fraction separation (e.g. cells) of donor blood, etc.

Further, the inventive monitoring techniques are applicable to any type of pumping device that generates pressure pulses in the first fluid containing system, not only rotary peristaltic pumps as disclosed above, but also other types of positive displacement pumps, such as linear peristaltic pumps, diaphragm pumps, as well as centrifugal pumps.

Tests have shown that different evaluation parameters may be preferable in different situations. For example, the use of variance or averaged sum of multiple pulses may increase the accuracy and/or detectability in various situations. Pattern recognition may be resorted when other detection methods fail.

Furthermore, although it may generally be enough for evaluation purposes to involve one parameter value, it may be advantageous to base an evaluation on combinations of two or more parameter values, such as to improve the reliability of the flow determination. The reliability may also be enhanced by increasing the evaluation time period. It may further be advantageous to increase the resolution in the measurements to further improve the reliability.

Although the model has been described with examples involving the heart pulses as primary source for analysis, the breathing of a subject may also be used as a primary source for analysis. As with heart pulses, breathing also constitutes a modulator of cardiovascular flow rates Q and similar parameters P may be used for measuring the for instance amplitude, shape, phase and/or frequency on the temporal signal profile.

### IX. SIGNAL EXTRACTION

In the following, embodiments for eliminating or reducing various pressure artefacts (also denoted "pump pulses" or "interference pulses") originating from one or more pulse generators in or associated with extracorporeal circuit will be described. Then, embodiments for isolating pressure data originating from a relevant physiological phenomenon among pressure pulses or pressure modulations originating from other physiological phenomena are described.

The pressure data to be extracted is not limited to a single physiological phenomenon and may originate from one or more physiological phenomena, including the heart. As used herein, the pressure data to be isolated is also denoted "subject pulses" or "patient pulses".

### Elimination of artefacts

Elimination of artefacts may be provided by:
- Controlling a pulse generator in the extracorporeal fluid system, such as a pump
   o By temporarily shutting down the pulse generator;
   o Shifting the frequency of the pulse generator;
- Low pass, band pass or high pass filtering;
- Spectral analysis and filtering in the frequency domain;
- Time domain filtering.

### Controlling a pulse generator

Artefacts from a pulse generator, such as a pump, in the extracorporeal fluid circuit may be avoided by temporarily shutting down (disabling) the pulse generator, or by shifting the frequency of the pulse generator away from frequencies of one or more relevant physiological phenomena.A feedback control with respect to the heart rate, e.g. obtained from a dedicated pulse sensor attached to the patient or obtained via analysis of previous parts of the monitoring signal, may be used to set the pump frequency optimally for detection of heart pulses. Similar feedback control may be used to eliminate artefacts with respect to pressure pulses originating from breathing, e.g. based on a breathing signal from an independent source, such as a capnograph instrument. Hence, the control unit 23 of Fig. 1 may be operated to control the pump frequency in order to facilitate the detection of the subject pulses, e.g. the pump frequency is controlled to minimize any overlap in frequency between the pump pulses and the subject pulses. For example, the pump frequency may be periodically increased and decreased around the overlap frequency, so as to maintain the overall blood flow rate. In a variant, the pump frequency is instead controlled so as to synchronize the rate of pump pulses with the rate of subject pulses while applying a phase difference between the pump pulses and the subject pulses. Thereby, the pump pulses and the subject pulses will be separated in time, and the subject pulses may be detected in the time domain, even without removal of the pump pulses. The phase difference may be approximately 180°, since this may maximize the separation of the pump pulses and the subject pulses in the time domain. This so-called phase-locking technique may be activated when it is detected that the rate of subject pulses approaches the rate of pump pulses, or vice versa.

### Applying low pass, band pass or high pass filters

The input signal to step 903 (Fig. 9) may be fed into a filter, e.g. digital or analogue, with frequency characteristics, such as frequency range and/or centre of frequency range, matched to the frequencies generated by a pulse generator, such as a pump, in the extracorporeal circuit. For instance, in a case where the pulse generator, such as a pump, operates within the frequency range of 1Hz, a suitable low pass filter may be applied in order to remove pressure artefacts above 1 Hz while retaining frequency components of the physiological phenomenon below 1 Hz. Correspondingly, a high pass filter may be applied to retain frequency components above the frequency of the the pulse generator. Alternatively, one or more notch filters or the like may be utilised to remove/attenuate frequencies in one or more confined ranges.

### Spectral analysis and filtering in the frequency domain

The input signal to part 133 may be subjected to a spectral analysis, e.g. by applying a Fourier transformation technique, such as FFT (Fast Fourier Transform) to convert the input signal into the frequency domain. The resulting energy spectrum (amplitude spectrum) may then be multiplied by an appropriate filter function and then re-transformed into the time domain. There are many alternative and equivalent filtering techniques available to the skilled person.

### Time domain filtering

Artefact elimination by filtering in the time domain is further disclosed and exemplified in Applicant's International patent publication WO2009/156175, entitled "Method and device for processing a time-dependent measurement signal".

### Isolating pressure data from a physiological phenomenon

Isolating pressure data originating from a relevant physiological phenomenon may be provided by any or a combination of:
- Low pass, band pass or high pass filtering;
- Spectral analysis and filtering in the frequency domain; or
- Time domain filtering.

### Applying low pass, band pass or high pass filters

The input signal to step 905 may be fed into a filter, e.g. digital or analogue, with frequency characteristics, such as frequency range and/or centre of frequency range, matched to the frequencies of pressure pulses from a relevant physiological phenomenon where e.g. in case the isolation concerns:
- Heart pulses, a frequency range substantially of 0.5 - 4 Hz will be allowed to pass the filter;
- Breathing, a frequency range substantially of 0.15 - 0.4 Hz will be allowed to pass the filter;
- Blood pressure regulation due to the autonomous system, a frequency range substantially of 0.04 - 0.15 Hz will be allowed to pass the filter; and
- Temperature regulation due to the autonomous system, a frequency range substantially of 0.001 - 0.1 Hz will be allowed to pass the filter.

### Spectral analysis and filtering in the frequency domain

The input signal to step 905 may be subjected to a spectral analysis, e.g. by applying a Fourier transformation technique, such as FFT (Fast Fourier Transform) to convert the input signal into the frequency domain. The resulting energy spectrum (amplitude spectrum) may then be multiplied by an appropriate filter function and then re-transformed into the time domain. There are many alternative and equivalent filtering techniques available to the skilled person.

### Time domain filtering

The signal of interest may be extracted from the input signal to step 905 as an error signal of an adaptive filter. The adaptive filter is fed with both the measured pressure signal and a predicted signal profile of a cyclic disturbance. The cyclic disturbance may originate from any unwanted physiological phenomenon (e.g. heart pulsation or breathing). Particularly, a reconstructed pressure profile originating from the unwanted physiological phenomenon may be input to the adaptive filter. This and other time domain filtering techniques for removing unwanted signal components from a measurement signal is further disclosed and exemplified in aforesaid WO2009/156175.

Some of the filtering techniques described above may automatically be achieved by down-sampling, since it may be taken care of by the anti-aliasing filter included in a down-sampling signal processing algorithm. Additionally, some of the above described filtering techniques may also be achieved directly in hardware, e.g., in the Analogue-to-Digital conversion by choosing an appropriate sample frequency, i.e. due to the anti-aliasing filter which is applied before sampling.

The term "comprises/comprising" when used in this specification is taken to specify the presence of stated features, integers, steps or components. However, the term does not preclude the presence or addition of one or more additional features, integers, steps or components or groups thereof.

The invention is not restricted to the described embodiments in the figures, but may be varied freely within the scope of the claims.

## Claims

1. A device for monitoring a fluid flow rate (Q) of a cardiovascular system of a subject, said device comprising an input (28) for obtaining a time-dependent measurement signal *(d(n))* from a pressure sensor (4a-4c) in an extracorporeal blood circuit (20) which is adapted for connection to the cardiovascular system, the pressure sensor (4a-4c) being arranged to detect a subject pulse originating from a subject pulse generator (3') in the cardiovascular system of the subject, wherein the device further comprises a signal processor (29) connected to the input (28) and being configured to:
process the measurement signal to obtain a pulse profile *(e(n))* which is a temporal signal profile of the subject pulse, and
calculate the fluid flow rate (Q) based at least partly on the temporal signal profile.

2. The device according to claim 1, wherein the subject pulse generator (3') is any of the heart, the breathing system, or any combinations thereof.

3. The device according to any of the preceding claims, wherein the signal processor (29) is further configured to calculate the fluid flow rate (Q) by involving one or more of an amplitude, a shape, and a timing of the temporal signal profile.

4. The device according to any of claims 1 to 3, wherein the signal processor (29) is further configured to aggregate a plurality of pulse profiles within an aggregation time window in the measurement signal and calculate the fluid flow rate (Q) based on an average of the plurality of the pulse profiles.

5. The device according to any of claims 1 to 4, wherein the signal processor (29) is configured to calculate the cardiac output (CO) of the cardiovascular system.

6. The device according to any of claims 1 to 5, wherein the signal processor (29) is configured to calculate an access flow (Qa) of a blood access in the cardiovascular system.

7. The device according to claim 1, wherein the signal processor (29) is further configured to calibrate the fluid flow rate (Q) against one or more calibration values.

8. The device according to any of claims 1 to 7, wherein the signal processor (29) is further configured to calculate an average access flow rate Qa and an associated variance QaV, retrieve a withdrawal blood flow rate Qb, and in case the sum of Qb and QaV exceeds Qa, generate an alarm event.

9. The device according to any of claims 1 to 8, wherein the signal processor (29) is further configured to:
calculate at least one additional fluid flow rate (Qx);
calculate an an average fluid flow rate (Qavg) determined from the calculated fluid flow rate (Q) and the at least one additional fluid flow rate (Qx);
calculate an average reference fluid flow rate (Qavg_ref); and
adjust the average reference fluid flow rate (Qavt_ref) based on the fluid flow rate (Q) and the at least oen additional fluid flow rate (Qx).

10. The device according to any of claims 1 to 9, wherein the signal processor (29) is further configured to calculate a reference fluid flow rate (Qref) at a time point when the calculated fluid flow rate (Q) corresponds to an average fluid flow rate.

11. The device according to claim 5, wherein an alarm event is generated when the Cardiac Output (CO) exceeds a predetermined threshold.

12. The device according to any of claims claim 1 to 11, wherein the signal processor (29) is further configured to:
define an initial model (M₀);
assign the initial model (M₀) to a current model (CM);
generate a parameter (P) that correlates with the fluid flow rate (Q);
acquire flow calibration data (C);
investigate whether a model validity criterion (MVC) is fulfilled or not by comparing the parameter (P) and the flow calibration data (C) with the current model (CM), wherein in case the model validity criterion (MVC) is not fulfilled then the signal processor (29) is configured to repeatedly generate a new model (NM) and assign the current model (CM) with the new model (NM) until the model validity criterion (MVC) is fulfilled;
wherein in case the model validity criterion (MVC) is fulfilled, the signal processor (29) is configured to calculate the fluid flow rate (Q) based at least partly on the temporal signal profile.

13. The device according to claim 12, wherein the signal processor (29) is further configured to perform one or more of:
acquiring blood pressure (BP) of the subject and comparing said blood pressure (BP) with the current model (CM); and
storing of the current model (CM) and available parameters (M, C, BP, P, TD, PD).

14. A method for monitoring a fluid flow rate (Q) in a cardiovascular system of a subject, said method comprising:
obtaining a time-dependent measurement signal *(d(n))* from a pressure sensor (4a-4c) in an extracorporeal blood circuit (20) which is arranged in fluid connection with the cardiovascular system, the pressure sensor (4a-4c) being arranged to detect a subject pulse originating from a subject pulse generator (3') in the cardiovascular system of the subject,
processing the measurement signal to obtain a pulse profile *(e(n))* which is a temporal signal profile of the subject pulse, and
calculating the fluid flow rate (Q) based at least partly on the temporal signal profile.

15. A computer-readable medium comprising computer instructions which, when executed by a processor, cause the processor to perform the method of claim 14.

## Patentansprüche

1. Vorrichtung zum Überwachen einer Strömungsrate (Q) eines Herz-Kreislauf-Systems eines Individuums, wobei die Vorrichtung einen Eingang (28) zum Erhalten eines zeitabhängigen Messsignals *(d(n))* von einem Drucksensor (4a bis 4c) in einem extrakorporalen Blutkreislauf (20) umfasst, welcher für eine Verbindung mit dem Herz-Kreislauf-System geeignet ist, wobei der Drucksensor (4a bis 4c) dafür eingerichtet ist, einen Puls des Individuums zu erfassen, der von einem Pulsgenerator (3') des Individuums in dem Herz-Kreislauf-System des Individuums stammt, wobei die Vorrichtung ferner einen Signalprozessor (29) umfasst, der mit dem Eingang (28) verbunden ist und für Folgendes konfiguriert ist:
Verarbeiten des Messsignals, um ein Pulsprofil *(e(n))* zu erhalten, welches ein zeitliches Signalprofil des Pulses des Individuums ist, und
Berechnen der Strömungsrate (Q) zumindest teilweise auf der Grundlage des zeitlichen Signalprofils.

2. Vorrichtung nach Anspruch 1, wobei der Pulsgenerator (3') des Individuums das Herz, das Atmungssystem oder eine beliebige Kombination davon ist.

3. Vorrichtung nach einem der vorhergehenden Ansprüche, wobei der Signalprozessor (29) ferner dafür konfiguriert ist, die Strömungsrate (Q) unter Einbeziehung einer Amplitude, einer Form und/oder eines Zeitverlaufs des zeitlichen Signalprofils zu berechnen.

4. Vorrichtung nach einem der Ansprüche 1 bis 3, wobei der Signalprozessor (29) ferner dafür konfiguriert ist, innerhalb eines Kumulationszeitfensters in dem Messsignal mehrere Pulsprofile zu kumulieren und die Strömungsrate (Q) auf der Grundlage eines Mittelwerts der mehreren Pulsprofile zu berechnen.

5. Vorrichtung nach einem der Ansprüche 1 bis 4, wobei der Signalprozessor (29) dafür konfiguriert ist, das Herzminutenvolumen (Cardiac Output, CO) des Herz-Kreislauf-Systems zu berechnen.

6. Vorrichtung nach einem der Ansprüche 1 bis 5, wobei der Signalprozessor (29) dafür konfiguriert ist, einen Zustrom (Qa) eines Blutzugangs in dem Herz-Kreislauf-System zu berechnen.

7. Vorrichtung nach Anspruch 1, wobei der Signalprozessor (29) ferner dafür konfiguriert ist, die Strömungsrate (Q) anhand eines oder mehrerer Kalibrierwerte zu kalibrieren.

8. Vorrichtung nach einem der Ansprüche 1 bis 7, wobei der Signalprozessor (29) ferner dafür konfiguriert ist, eine mittlere Zustromrate Qa und eine zugehörige Varianz QaV zu berechnen, eine Blutentnahme-Strömungsrate Qb abzurufen und in dem Fall, dass die Summe aus Qb und QaV Qa übersteigt, ein Alarmereignis zu erzeugen.

9. Vorrichtung nach einem der Ansprüche 1 bis 8, wobei der Signalprozessor (29) ferner für Folgendes konfiguriert ist:
Berechnen mindestens einer weiteren Strömungsrate (Qx);
Berechnen einer mittleren eine Zugangsströmung (Qavg), die aus der berechneten Strömungsrate (Q) und der mindestens einen weiteren Strömungsrate (Qx) bestimmt wird;
Berechnen einer mittleren Referenzströmungsrate (Qavg_ref) und
Anpassen der mittleren Referenzströmungsrate (Qavg_ref) auf der Grundlage der Strömungsrate (Q) und der mindestens einen weiteren Strömungsrate (Qx).

10. Vorrichtung nach einem der Ansprüche 1 bis 9, wobei der Signalprozessor (29) ferner dafür konfiguriert ist, eine Referenzströmungsrate (Qref) zu einem Zeitpunkt zu berechnen, wenn die berechnete Strömungsrate (Q) einer mittleren Strömungsrate entspricht.

11. Vorrichtung nach Anspruch 5, wobei ein Alarmereignis erzeugt wird, wenn das Herzminutenvolumen (Cardiac Output, CO) einen vorgegebenen Schwellenwert übersteigt.

12. Vorrichtung nach einem der Ansprüche 1 bis 11, wobei der Signalprozessor (29) ferner für Folgendes konfiguriert ist:
Definieren eines Anfangsmodells (M₀);
Zuordnen des Anfangsmodells (M₀) zu einem aktuellen Modell (Current Model, CM);
Erzeugen eines Parameters (P), welcher mit der Strömungsrate (Q) in Korrelation steht;
Erhalten von Strömungskalibrierdaten (C);
Untersuchen, ob ein Modellgültigkeitskriterium (Model Validity Criterion, MVC) erfüllt ist oder nicht, durch Vergleichen des Parameters (P) und der Strömungskalibrierdaten (C) mit dem aktuellen Modell (CM), wobei in dem Fall, dass das Modellgültigkeitskriterium (MVC) nicht erfüllt ist, der Signalprozessor (29) dann dafür konfiguriert ist, wiederholt ein neues Modell (NM) zu erzeugen und das neue Modell (NM) dem aktuellen Modell (CM) zuzuordnen, bis das Modellgültigkeitskriterium (MVC) erfüllt ist;
wobei in dem Fall, dass das Modellgültigkeitskriterium (MVC) erfüllt ist, der Signalprozessor (29) dafür konfiguriert ist, die Strömungsrate (Q) zumindest teilweise auf der Grundlage des zeitlichen Signalprofils zu berechnen.

13. Vorrichtung nach Anspruch 12, wobei der Signalprozessor (29) ferner dafür konfiguriert ist, eines oder mehreres aus dem Folgenden durchzuführen:
Erhalten eines Blutdrucks (Blood Pressure, BP) des Individuums und Vergleichen des Blutdrucks (BP) mit dem aktuellen Model (CM) und
Speichern des aktuellen Modells (CM) und verfügbarer Parameter (M, C, BP, P, TD, PD).

14. Verfahren zum Überwachen einer Strömungsrate (Q) in einem Herz-Kreislauf-System eines Individuums, wobei das Verfahren Folgendes umfasst:
Erhalten eines zeitabhängigen Messsignals (d(n)) von einem Drucksensor (4a bis 4c) in einem extrakorporalen Blutkreislauf (20), welcher in Fluidverbindung mit dem Herz-Kreislauf-System angeordnet ist, wobei der Drucksensor (4a bis 4c) dafür eingerichtet ist, einen Puls des Individuums zu erfassen, der von einem Pulsgenerator (3') des Individuums in dem Herz-Kreislauf-System des Individuums stammt,
Verarbeiten des Messsignals, um ein Pulsprofil *(e(n))* zu erhalten, welches ein zeitliches Signalprofil des Pulses des Individuums ist, und
Berechnen der Strömungsrate (Q) zumindest teilweise auf der Grundlage des zeitlichen Signalprofils.

15. Computerlesbares Medium, umfassend Computerbefehle, welche, wenn sie von einem Prozessor ausgeführt werden, bewirken, dass der Prozessor das Verfahren nach Anspruch 14 durchführt.

## Revendications

1. Dispositif de contrôle d'une vitesse d'écoulement des fluides (Q) du système cardiovasculaire d'un sujet, ledit dispositif comprenant une entrée (28) pour obtenir un signal de mesure dépendant du temps *(d(n))* à partir d'un capteur de pression (4a-4c) présent dans un circuit sanguin extracorporel (20) adapté pour se connecter au système cardiovasculaire, le capteur de pression (4a-4c) étant agencé pour détecter une impulsion du sujet en provenance d'un générateur d'impulsions du sujet (3') présent dans le système cardiovasculaire du sujet, le dispositif comprenant en outre un processeur de signaux (29) connecté à l'entrée (28) et configuré pour :
traiter le signal de mesure pour obtenir un profil d'impulsion *(e(n))* qui est un profil de signal temporel de l'impulsion du sujet, et
calculer la vitesse d'écoulement des fluides (Q) en se fondant au moins en partie sur le profil de signal temporel.

2. Dispositif selon la revendication 1, dans lequel le générateur d'impulsions du sujet (3') est le coeur, le système respiratoire ou l'une quelconque de leurs combinaisons.

3. Dispositif selon l'une quelconque des revendications précédentes, dans lequel le processeur de signaux (29) est en outre configuré pour calculer la vitesse d'écoulement des fluides (Q) en utilisant une amplitude, une forme et/ou un moment du profil de signal temporel.

4. Dispositif selon l'une quelconque des revendications 1 à 3, dans lequel le processeur de signaux (29) est en outre configuré pour agréger une pluralité de profils d'impulsion dans une fenêtre de temps d'agrégation dans le signal de mesure et calculer la vitesse d'écoulement des fluides (Q) à partir d'une moyenne de la pluralité des profils d'impulsion.

5. Dispositif selon l'une quelconque des revendications 1 à 4, dans lequel le processeur de signaux (29) est configuré pour calculer le débit cardiaque (CO) du système cardiovasculaire.

6. Dispositif selon l'une quelconque des revendications 1 à 5, dans lequel le processeur de signaux (29) est configuré pour calculer un écoulement d'accès (Qa) d'un accès de sang dans le système cardiovasculaire.

7. Dispositif selon la revendication 1, dans lequel le processeur de signaux (29) est en outre configuré pour étalonner la vitesse d'écoulement des fluides (Q) par rapport à une ou plusieurs valeurs d'étalonnage.

8. Dispositif selon l'une quelconque des revendications 1 à 7, dans lequel le processeur de signaux (29) est en outre configuré pour calculer une vitesse moyenne d'écoulement d'accès Qa et une variance associée QaV, récupérer une vitesse d'écoulement de sang de retrait Qb et, lorsque la somme de Qb et QaV dépasse Qa, générer un évènement d'alarme.

9. Dispositif selon l'une quelconque des revendications 1 à 8, dans lequel le processeur de signaux (29) est en outre configuré pour :
calculer au moins une vitesse d'écoulement des fluides supplémentaire (Qx) ;
calculer une vitesse moyenne d'écoulement des fluides (Qavg) déterminée à partir de la vitesse d'écoulement des fluides (Q) calculée et de la ou des vitesses d'écoulement des fluides supplémentaires (Qx) ;
calculer une vitesse moyenne d'écoulement des fluides de référence (Qavg_ref) ; et
ajuster la vitesse moyenne d'écoulement des fluides de référence (Qavg_ref) à partir de la vitesse d'écoulement des fluides (Q) et de la ou des vitesses d'écoulement des fluides supplémentaires (Qx).

10. Dispositif selon l'une quelconque des revendications 1 à 9, dans lequel le processeur de signaux (29) est en outre configuré pour calculer une vitesse d'écoulement des fluides de référence (Qref) à un moment où la vitesse d'écoulement des fluides (Q) calculée correspond à une vitesse moyenne d'écoulement des fluides.

11. Dispositif selon la revendication 5, dans lequel un évènement d'alarme est généré lorsque le débit cardiaque (CO) dépasse un seuil prédéterminé.

12. Dispositif selon l'une quelconque des revendications 1 à 11, dans lequel le processeur de signaux (29) est en outre configuré pour :
définir un modèle initial (M₀) ;
attribuer le modèle initial (M₀) à un modèle actuel (CM) ;
générer un paramètre (P) qui est corrélé avec la vitesse d'écoulement des fluides (Q) ;
acquérir des données d'étalonnage de l'écoulement (C) ;
étudier si un critère de validité du modèle (MVC) est satisfait ou non en comparant le paramètre (P) et les données d'étalonnage de l'écoulement (C) avec le modèle actuel (CM) ; le processeur de signaux (29) étant configuré, lorsque le critère de validité du modèle (MVC) n'est pas satisfait, pour générer de manière répétée un nouveau modèle (NM) et attribuer le modèle actuel (CM) au nouveau modèle (NM) jusqu'à ce que le critère de validité du modèle (MVC) soit satisfait ;
le processeur de signaux (29) étant configuré, lorsque le critère de validité du modèle (MVC) est satisfait, pour calculer la vitesse d'écoulement des fluides (Q) en se fondant au moins en partie sur le profil de signal temporel.

13. Dispositif selon la revendication 12, dans lequel le processeur de signaux (29) est en outre configuré pour réaliser :
une acquisition de la tension artérielle (BP) du sujet et une comparaison de ladite tension artérielle (BP) avec le modèle actuel (CM) ; et/ou
un stockage du modèle actuel (CM) et des paramètres disponibles (M, C, BP, P, TD, PD).

14. Procédé de contrôle d'une vitesse d'écoulement des fluides (Q) dans le système cardiovasculaire d'un sujet, ledit procédé comprenant :
l'obtention d'un signal de mesure dépendant du temps *(d(n))* à partir d'un capteur de pression (4a-4c) présent dans un circuit sanguin extracorporel (20) agencé en connexion fluidique avec le système cardiovasculaire, le capteur de pression (4a-4c) étant agencé pour détecter une impulsion du sujet en provenance d'un générateur d'impulsions du sujet (3') présent dans le système cardiovasculaire du sujet,
le traitement du signal de mesure pour obtenir un profil d'impulsion (*e(n))* qui est un profil de signal temporel de l'impulsion du sujet, et
le calcul de la vitesse d'écoulement des fluides (Q) en se fondant au moins en partie sur le profil de signal temporel.

15. Support lisible par ordinateur, comprenant des instructions informatiques qui, lorsqu'elles sont exécutées par un processeur, provoquent la réalisation par le processeur du procédé selon la revendication 14.
